# EUROPEAN PATENT APPLICATION

(11) **EP 4 556 028 A1**
(43) Date of publication of application: **21.05.2025**
(21) Application number: 23209601.6
(22) Date of filing: 14.11.2023
(51) Int. Cl.: A61K 47/69, A61K 47/62, A61K 9/51, C07K 14/00, C07K 14/465, A61K 9/00, G01N 33/569, A61P 31/14

(54) **FUNCTIONALIZED NANOPARTICLES FOR VIRAL CLEARANCE**

(71) Applicant: Leibniz-Institut für Naturstoff-Forschung und Infektionsbiologie - Hans-Knöll-Institut -, 07745 Jena (DE); Friedrich-Schiller-Universität Jena, 07737 Jena (DE); Julius-Maximilians-Universität Würzburg, 97070 Würzburg (DE); Leibniz-Institut für Virologie, 20251 Hamburg (DE)
(72) Inventor: Brakhage, Axel, 99425 Weimar (DE); Kloß, Florian, 4055 Basel (CH); Hortschansky, Peter, 07749 Jena (DE); Preusske, Nils, 24106 Kiel (DE); Schmidt, Franziska, 99085 Erfurt (DE); Schubert, Ulrich, 07743 Jena (DE); Schubert, Stephanie, 07743 Jena (DE); Behnke, Mira, 44532 Lünen (DE); Adermann, Franziska, 07743 Jena (DE); Backes, Simone, 97082 Würzburg (DE); Gasteiger, Georg, 97082 Würzburg (DE); Gabriel, Gülsah, 22587 Hamburg (DE); Beck, Sebastian, 22523 Hamburg (DE)
(74) Representative: Krauss, Jan

(57) **Abstract**

The present invention relates to functionalized nano- or microparticles of suitable size and shape, comprising a nano- or microparticle and at least one virus-binding peptide and/or virus-binding small molecule immobilized onto the surface of the nano- or microparticle. These nano- or microparticle forms aggregates with targeted viral particles to initiate phagocytosis, thereby achieving viral clearance. The present invention further relates to uses of the functionalized nano- or microparticles in virus detection, therapy and diagnosis.

## Description

The present invention relates to functionalized nano- or microparticles of suitable size and shape, comprising a nano- or microparticle and at least one virus-binding peptide and/or virus-binding small molecule immobilized onto the surface of the nano- or microparticle. These nano- or microparticle forms aggregates with targeted viral particles to initiate phagocytosis, thereby achieving viral clearance. The present invention further relates to uses of the functionalized nano- or microparticles in virus detection, therapy and diagnosis.

### Background of the invention

Viral infectious diseases are an important factor both in human and veterinary healthcare. Transmission, spread and especially pathophysiology of viruses are highly pathogen specific and strongly depend on the adaptation of virus particles to the host, making it challenging to develop targeted therapies that are effective across viral strains.

Particularly airborne viruses can be highly infectious and sometimes lead to threatening exponential dissemination including larger outbreaks and even pandemics. A prominent example is the global pandemic associated with SARS-CoV-2 which belongs to the family of coronaviruses. Betacoronaviruses (β-CoVs or Beta-CoVs) are one of four genera of coronaviruses of the subfamily *Orthocoronavirinae* in the family *Coronaviridae,* of the order *Nidovirales.* They are enveloped, positive-sense, single-stranded RNA viruses of zoonotic origin. The need for viral treatment, prevention and diagnostics is ever-growing.

Some liposome-based viral therapies are available. A virosome-formulated vaccine, Epaxal^{®}, based on formalin inactivated HAV (strain RG-SB) adsorbed to the surface of special liposomes (virosomes) has been developed (Bovier PA. Epaxal: a virosomal vaccine to prevent hepatitis A infection. Expert Rev Vaccines. 2008;7(8):1141-1150. doi:10.1586/14760584.7.8.1141). Also, Inflexal^{®} a virosomal (150 nm liposomes) vaccine mimicking native antigen presentation has been on the market for some time. Here, liposomes mimic the native virus structure, thus allowing for cellular entry and membrane fusion (Retention of the natural presentation of antigens on liposomal surface provides for high immunogenicity. Herzog C, Hartmann K, Künzi V, et al. Eleven years of Inflexal V-a virosomal adjuvanted influenza vaccine. Vaccine. 2009;27(33):4381-4387. doi:10.1016/j.vaccine.2009.05.029).

Singh et al. (in: The role of nanotechnology in the treatment of viral infections. Ther Adv Infect Dis. 2017;4(4):105-131. doi:10.1177/2049936117713593) present a broad overview of the application of nanosized materials for the treatment of common viral infections.

Virus-mimetic cell interactive nanoparticles (NPs) are described as another valuable strategy to enhance NP specificity for therapeutic and diagnostic applications (Maslanka Figueroa S, et al. Influenza A virus mimetic nanoparticles trigger selective cell uptake. Proc Natl Acad Sci U S A. 2019;116(20):9831-9836. doi:10.1073/pnas. 1902563116).

US 2013-0337066 describes a nanoparticle comprising: a) an inner core comprising a non-cellular material; and b) an outer surface comprising a cellular membrane derived from a cell or a membrane derived from a virus. The inner core may comprise a biocompatible or a synthetic material selected from the group consisting of poly(lactic-co-glycolic acid) (PLGA), polylactic acid (PLA), polyglycolic acid (PGA), polycaprolactone (PCL), polylysine, and polyglutamic acid, and the cellular membrane is derived from a blood cell, a tumor cell, a cancer cell, an immune cell, a stem cell, an endothelial cell, an exosome, a secretory vesicle or a synaptic vesicle. The nanoparticle may comprise a releasable cargo, such as a therapeutic agent, a prophylactic agent, a diagnostic or marker agent, a prognostic agent, or a combination thereof.

During their infection and proliferation, pathogenic viruses often utilize recognition sites (receptor domains) of their host cells to hijack intracellular uptake mechanisms, such as micropinocytosis and clathrin-mediated or -independent endocytosis. Intracellular uptake is essential for the infection process of viruses, as their proliferation intrinsically relies on cellular machineries such as the ribosomal complex in order to provide all building blocks and enzymes required for the assembly and release of viral particles. While current antiviral treatments mainly involve targeting of intracellular viral enzymatic pathways, only few compounds actively and efficiently prevent the entry of viruses (e.g. enfuvirtide).

A very promising concept to inhibit the internalization of viruses is the competitive saturation of viral envelope proteins (e.g. spike proteins like for SARS-CoV-2) essential for host cell contact to prevent effective protein-protein-interaction. However, saturation of the viral envelope may only decelerate the infection process as viruses are not actively neutralized through the immune system.

Cagno et al. (in: Broad-Spectrum Non-Toxic Antiviral Nanoparticles with a Virucidal Inhibition Mechanism. Nat. Mater. 2018, 17, 195-203. 10.1038/nmat5053) designed antiviral nanoparticles with long and flexible linkers mimicking HSPG, allowing for effective viral association with a binding that the inventors simulate to be strong and multivalent to the VAL repeating units, generating forces (~190 pN) that eventually lead to irreversible viral deformation. They are active ex vivo in human cervicovaginal histocultures infected by HSV-2 and in vivo in mice infected with RSV.

Han et al. (in: Han Y, Král P. Computational Design of ACE2-Based Peptide Inhibitors of SARS-CoV-2. ACS Nano. 2020;14(4):5143-5147. doi:10.1021/acsnano.0c02857) describe potential peptide inhibitors against the SARS-CoV-2 coronavirus mostly formed by two sequential self-supporting α-helices (bundle) extracted from the protease domain (PD) of angiotensin-converting enzyme 2 (ACE2), which bind to the SARS-CoV-2 receptor binding domains. Molecular dynamics simulations revealed that the α-helical peptides maintain their secondary structure and provide a highly specific and stable binding (blocking) to SARS-CoV-2. The proposed small peptides ("minibinders") could be used as inhaled therapeutics for topical lung delivery, providing an efficient way to combat COVID-19. They further describe that in the early attempts of SARS-CoV blocking, short peptide inhibitors were studied, and amino acid mutations were implemented to the S protein of SARS-CoV. However, the proposed peptide was too short (8 residues) to maintain secondary structure, so it was unable to block the whole SARS-CoV binding surface. Broad-spectrum antiviral nanoparticles and cyclodextrins were designed, simulated, and implemented in blocking of other viruses. They are category 2 or 3 inhibitors, but their applicability to SARS-CoV-2 is unknown.

Han et al. propose that in order to provide a multivalent binding to the SARS-CoV-2 receptors, many such peptides could be attached to the surfaces of nanoparticle carriers. Nevertheless, these nanoparticles, dendrimers, and clusters are only designed in order to improve binding to the inhibitor.

APEIRON Biologics AG started the further development of APN01 for the treatment of severely ill COVID-19 patients; the soluble APN01 imitates the receptor ACE2 and thus offers a dual approach to treatment.

Cao L, et al. (in: De novo design of picomolar SARS-CoV-2 miniprotein inhibitors. Science. 2020 Oct 23;370(6515):426-431. doi: 10.1126/science.abd9909. Epub 2020 Sep 9. PMID: 32907861) describe that targeting the interaction between the severe acute respiratory syndrome coronavirus 2 (SARS-CoV-2) spike protein and the human angiotensin-converting enzyme 2 (ACE2) receptor is a promising therapeutic strategy. They designed inhibitors using two de novo design approaches. Computer-generated scaffolds were either built around an ACE2 helix that interacts with the spike receptor binding domain (RBD) or docked against the RBD to identify new binding modes, and their amino acid sequences were designed to optimize target binding, folding, and stability. Ten designs bound the RBD, with affinities ranging from 100 picomolar to 10 nanomolar, and blocked SARS-CoV-2 infection of Vero E6 cells with median inhibitory concentration (IC₅₀) values between 24 picomolar and 35 nanomolar. The most potent, with new binding modes, are 56- and 64-residue proteins (IC₅₀ ~ 0.16 nanograms per milliliter). Cryo-electron microscopy structures of these minibinders in complex with the SARS-CoV-2 spike ectodomain trimer with all three RBDs bound are nearly identical to the computational models. These hyperstable minibinders provide starting points for SARS-CoV-2 therapeutics.

Case et al. (in: Ultrapotent miniproteins targeting the SARS-CoV-2 receptor-binding domain protect against infection and disease. Cell Host Microbe. 2021 Jul 14;29(7):1151-1161.e5. doi: 10.1016/j.chom.2021.06.008. Epub 2021 Jun 24. PMID: 34192518) describe the capacity of modified versions of one lead miniprotein, LCB1, to protect against SARS-CoV-2-mediated lung disease in mice. Systemic administration of LCB1-Fc reduced viral burden, diminished immune cell infiltration and inflammation, and completely prevented lung disease and pathology. A single intranasal dose of LCB1v1.3 reduced SARS-CoV-2 infection in the lung when given as many as 5 days before or 2 days after virus inoculation. Importantly, LCB 1v1.3 protected in vivo against a historical strain (WA1/2020), an emerging B.1.1.7 strain, and a strain encoding key E484K and N501Y spike protein substitutions. These data support development of LCB1v1.3 for prevention or treatment of SARS-CoV-2 infection.

Despite advances in anti-viral therapy and vaccination, still effective and reliable agents and methods for the prevention and therapy of viral infection are sought that are also unlikely to develop viral resistance, and are well-tolerated by the patients. It is therefore an object of the present invention to provide effective agents that can be used for the prevention and treatment of viral infection, in particular coronavirus infection. Other objects and advantages will become apparent to the person of skill when studying the present description of the present invention.

In a first aspect thereof, the above object is solved according to the present invention by a functionalized nano- or microparticle for use in the prevention and/or treatment of target virus infection in a mammalian subject, comprising, a) at least one nano- or microparticle, and b) at least one virus-binding peptide and/or virus-binding small molecule immobilized onto the surface of the nano- or microparticle, wherein the virus-binding peptide and/or a virus-binding small molecule binds to the at least one target virus with nano- to picomolar affinity, wherein the functionalized nano- or microparticle has an overall diameter of at least about 400 nm, preferably between about 400 to 2000 nm, and more preferably between about 600 and 700 nm. The invention shall provide overall complexes that allow active phagocytosis, this requires a size of the particle of about > 400 nm.

Preferred is the functionalized nano- or microparticle for use according to the present invention, wherein said functionalized nano- or microparticle is substantially spherical in shape.

Further preferred is the functionalized nano- or microparticle for use according to the present invention, wherein the virus-binding peptide is selected from a minibinder protein, a host cell receptor, sialylated glycans such as SAs and SA-containing gangliosides, cell adhesion molecules (CAMs), such as igSF members including CD4, JAM-A, CAR, integrins, such as αvβ3, PtdSer receptors, cell immunoglobulin and mucin domain (TIM), Tyro3, Axl, Mer (TAM), and ACE2 receptor, preferably wherein the virus-binding peptide is selected from LCB 1, and AHB2.

In a second aspect thereof, the above object is solved according to the present invention by an aggregate comprising at least one functionalized nano- or microparticle for use according to the present invention and at least one target virus for use in the prevention and/or treatment of target virus infection in a mammalian subject, wherein said prevention and/or treatment comprises phagocytosis by cells, in particular macrophage mediated phagocytosis in said mammalian subj ect.

Preferred is the functionalized nano- or microparticle for use according to the present invention or the aggregate for use according to the present invention, wherein said at least one target virus is selected from the group of HIV, measles virus, reovirus, rhinovirus, adenovirus, poliovirus, coxsackievirus B, reovirus, rotavirus, adenovirus, West Nile virus, human metapneuomovirus (hMPV), foot-and-mouth disease virus (FMDV), herpes simplex virus (HSV), HPV, human cytomegalovirus HCMV, human herpesvirus-8, rabies virus, hMPV, paramyxovirus, filoviruses, Ebola virus (EBOV), Marburgvirus (MARV), flaviviruses, dengue virus (DENV), Zika virus (ZIKV), Lassa Virus, poxvirus, SV40, BKPyV, PyV, Influenza A virus (IAV), Orthomyxoviridae, MERS-CoV, betacoronavirus, SARS-S, and SARS-CoV-2.

In a third aspect thereof, the above object is solved according to the present invention by a pharmaceutical composition comprising the functionalized nano- or microparticle for use according to the present invention or the aggregate for use according to the present invention for use in the prevention and/or treatment of target virus infection in a mammalian subject, wherein said pharmaceutical composition additionally comprises suitable excipients and/or auxiliary agents.

Preferred is the pharmaceutical composition for according to the present invention, wherein said composition is formulated for intravenous injection, a drinking solution/suspension, a solution/suspension suitable for aerosol production and inhalation, or dry powder for inhalation. More preferred is the pharmaceutical composition for use according to the present invention, wherein said functionalized nano- or microparticle has size between about 400 to 700 nm and is optionally suitable for administration through inhalation.

In a fourth aspect thereof, the above object is solved according to the present invention by a method for detecting a target virus and/or target virus specific anti-virus antibodies in a biological sample obtained from a mammalian subject, comprising contacting the functionalized nano- or microparticle for use according to the present invention with said biological sample, and detecting binding of said target virus and/or target virus specific anti-virus antibodies to said particle and/or detecting forming of aggregates of said target virus with said functionalized nano- or microparticle, wherein a binding and/or forming of aggregates is indicative for the target virus and/or target virus specific anti-virus antibodies in the biological sample.

Preferred is the method according to the present invention, further comprising the step of quantifying the amount of a target virus and/or target virus specific anti-virus antibodies in the biological sample obtained from the mammalian subject based on the binding and/or forming of aggregates in the biological sample.

In a fifth aspect thereof, the above object is solved according to the present invention by a kit for performing the method according to the present invention, comprising at least one functionalized nano- or microparticle for use according to the present invention, together with suitable auxiliary agents.

In a sixth aspect thereof, the above object is solved according to the present invention by the use of the kit according to the present invention for detecting or quantifying a target virus and/or target virus specific anti-virus antibodies in a biological sample obtained from a mammalian subj ect.

In a seventh aspect thereof, the above object is solved according to the present invention by a method for preventing and/or treating a target virus infection in a mammalian subject, comprising administering the functionalized nano- or microparticle for use according to the present invention, the aggregate for use according to the present invention, or the pharmaceutical composition for use according to the present invention to a mammalian subject in need of preventing and/or treating a target virus infection.

Preferred is the method according to the present invention, wherein the target virus is selected from the group of HIV, measles virus, reovirus, rhinovirus, adenovirus, poliovirus, coxsackievirus B, reovirus, rotavirus, adenovirus, West Nile virus, human metapneuomovirus (hMPV), foot-and-mouth disease virus (FMDV), herpes simplex virus (HSV), HPV, human cytomegalovirus HCMV, human herpesvirus-8, rabies virus, hMPV, paramyxovirus, filoviruses, Ebola virus (EBOV), Marburgvirus (MARV), flaviviruses, dengue virus (DENV), Zika virus (ZIKV); Lassa Virus, poxvirus, SV40, BKPyV, PyV, Influenza A virus (IAV), Orthomyxoviridae, MERS-CoV, betacoronavirus, SARS-S, and SARS-CoV-2.

As mentioned above, the present invention provides a functionalized nano- or microparticle for use in the prevention and/or treatment of target virus infection in a mammalian subject. The at least one nano- or microparticle is functionalized with at least one virus-binding peptide and/or virus-binding small molecule that is/are immobilized onto the surface of the nano- or microparticle. These virus-binding peptides and/or a virus-binding small molecules bind to the at least one target virus with nano- to picomolar affinity. Finally, the functionalized nano- or microparticle has an overall wherein the functionalized nano- or microparticle has an overall diameter of at least about 400 nm, preferably between about 400 to 2000 nm, and more preferably between about 600 and 700 nm. Typically, nano- or microparticles are recognized by the host immune system and potentially removed by phagocytosis in sizes of between about 100 to about 10,000 nm.

In a particularly preferred embodiment of the invention, the functionalized nano- or microparticle for use has size between about 400 to 700 nm and is optionally suitable for administration to a mammalian subject through inhalation.

Behbahanipour M, et al. (in: OligoBinders: Bioengineered Soluble Amyloid-like Nanoparticles to Bind and Neutralize SARS-CoV-2. ACS Appl Mater Interfaces. 2023 Mar 8;15(9):11444-11457. doi: 10.1021/acsami.2c18305. Epub 2023 Feb 22. PMID: 36890692; PMCID: PMC9969896) describe a kind of nanoparticle that can neutralize SARS-CoV-2. To this purpose, we exploited a modular self-assembly strategy to engineer OligoBinders, soluble oligomeric nanoparticles decorated with two miniproteins previously described to bind to the S protein receptor binding domain (RBD) with high affinity. The multivalent nanostructures compete with the RBD-ACE2r interaction and neutralize SARS-CoV-2 virus-like particles (SC2-VLPs) with IC₅₀ values in the pM range, preventing SC2-VLPs fusion with the membrane of ACE2r-expressing cells. Moreover, OligoBinders are biocompatible and significantly stable in plasma. The particles as used included the peptides LCB 1, and have a size of about 20 nm.

In contrast, the particles used according to the invention actively use phagocytosis, this requires a size of the complex of more than about 400 nm - if the particles are too small, they can only bind a limited amount of viral particles. This impairs the neutralization or requires a higher dosage of the nanoparticles. Furthermore, the detectability in cells and tissues is decisive in order to determine the therapeutic functionality in vivo as well as in vitro. In case of complexes having a size of less than 400nm, this becomes substantially more difficult.

Little particles are able to cross the mucosal barrier, and thus get lost. This shall be avoided in the context of the present invention. If the particles cross or get into the mucosal barrier after application, the particle/virus contact is inhibited/obstructed. This results in a reduced therapeutic effect. The use of a PEG-linker also increases the mucosal crossing, therefore, particles of a size of about 600-700 nm are preferred.

Finally, markedly larger particles of more than 900 nm trigger an adverse immune reaction, which in the context of peptide-decoration and the later viral interaction is not desirable.

In the context of the present invention, the nano- or microparticle may be made of any material suitable for administration to a subject and/or suitable for the diagnostic use as disclosed herein. The material also has to be suitable to provide a functionalized particle. In the context of the present invention, the term "functionalized" shall refer to a modification of the surface of the particle to be used in the methods according to the present invention. According to the invention, the functionalization of the particle comprises at least one virus-binding peptide and/or virus-binding small molecule that is/are immobilized onto the surface of the nano- or microparticle.

In a preferred embodiment of the present invention, the choice regarding the combination of material comprising the nano- or microparticle and the virus-binding peptide and/or virus-binding small molecule can be coordinated as desired, i.e. a modular design of the functionalized nano- or microparticle may be achieved. In this modular design according to the present invention, a polymeric organic or inorganic material compatible for the desired route of administration and functionalization is selected to make up the nano- or microparticle. Preferably, particle materials are used which are sufficiently stable towards host metabolism, but not fully inert to enable sufficient clearance.

Particle selection may further be driven by toxicological parameters and local tolerance. This polymeric material may be, for example, PLA, PLGA, PLGH, polyglutamate, styrene-maleic acid copolymer or PE, or is a nanosphere composed of membranes and/or lipids, such as a liposome.

In a preferred embodiment of the present invention, the polymeric material comprises poly(lactic-co-glycolic acid) (PLGA), which is often used due to its well documented biocompatibility and biodegradability. The monomers lactide acid and glycolic acid can be metabolized by the body, and by varying the monomer ratios it is possible to influence the degradation rate. In another preferred embodiment of the present invention, the polymer material additionally comprises a blockcopolymer comprising PLGA and poly(ethylene glycol) (PEG) as a stealth polymer. Upon formulation, the hydrophilic PEG should be located towards the outside of the particle, thus, the ω-end groups should be found on the surface of the particle facilitating any surface modification.

In a related embodiment of the present invention, the polymeric material comprises PLGA-Mal, PLGA-PEG-Mal, PLGA-DY550, PLGA-PEG, PLGA-PEG-DBCO, and/or a combination thereof. In another preferred embodiment of the present invention, the nano- or microparticle comprises any polymer composition according to Table 1.

**Table 1: Preferred polymer compositions.**

| **Polymers** | **Polymer ratio** |
|---|---|
| PLGA-Mal | 1 |
| PLGA-PEG-Mal | 1 |
| PLGA-Mal / PLGA | 1:1 |
| PLGA-PEG-Mal / PLGA | 1:1 |
| PLGA-Mal / PLGA | 1:9 |
| PLGA-PEG-Mal / PLGA | 1:9 |
| PLGA | 1 |
| PLGA-Mal/PLGA-DY550 | 1:1 |
| PLGA-PEG-Mal / PLGA | 1:1 |
| PLGA | 1 |
| PLGA-PEG / PLGA | 1:1 |
| PLGA-PEG-DBCO / PLGA | 1:1 |
| PLGA-PEG / PLGA | 1:1 |

Preferably, the nano- or microparticle comprises a suitable organic or inorganic material, such as, for example, a metal, a plastic, a polymer, such as, for example, PLA, PLGA, PLGH, polyglutamate, styrene-maleic acid copolymer or PE, or is a nanosphere composed of membranes and/or lipids, such as a liposome, preferably wherein said nano- or microparticle comprises PLGA-Mal, PLGA-PEG-Mal, PLGA-DY550, PLGA-PEG, PLGA-PEG-DBCO, and/or combinations thereof.

In addition to a certain functionalization and the size of the functionalized nano- or microparticle for use according to the present invention, the shape of the functionalized nano- or microparticle was found to be relevant for its use. The particle geometry can help in modulating the cellular internalization of the particle or aggregate (see below) via phagocytosis. Different local particle shapes at the point of cell attachment generates different angles between the membrane and particle. This contact angle has a significant effect on the ability of macrophages to internalize particles via actin-driven movement of the macrophage membrane. Preferably, the particle is substantially spherical in shape, in contrast to, for example, oblate ellipsoids, prolate ellipsoids, elliptical disks, rectangular disks, and UFOs. Elongated particles are known to be less prone to phagocytosis.

Another important feature of the present invention, in contrast the state of the art, is the highly specific binding of the virus-binding peptides and/or a virus-binding small molecules on the surface of the nano- or microparticle to the target virus. In particular embodiments, the functionalized nano- or microparticle competes with the host cell receptor for binding to the virus, in order to a) prevent infection-relevant interactions with the native host cell receptors and b) trigger the uptake through macrophages.

Upon functionalization of the nano- or microparticles with the present high affinity virus-binding peptides and/or virus-binding small molecules, viruses can selectively be captured and made directly accessible towards macrophage mediated phagocytosis. A particularly preferred embodiment of the present invention is therefore the at least nano- to picomolar range binding between the virus-binding peptides and/or a virus-binding small molecules and the target virus.

The use of structural entities that bind the target virus particle with nanomolar to picomolar affinity is advantageous since it allows the nano- or microparticles to outcompete the native receptor for binding of virions. For example, in the case of the SARS-CoV-2 spike protein, the virus binder on the surface of nanoparticles needs to be able to interact with the wild-type (wt) spike RBD with nanomolar affinity, to outcompete the native ACE2 receptor.

The virus-binding small molecule can be selected from small molecule virus binders such as elbasvir, grazoprevir, sovaprevir, hesperidin, pamaqueside, diosmin, and sitogluside, and the person skilled in the art is aware of many more different high affinity binders to viral particles in the state of the art.

The virus-binding peptide can be selected from a minibinder protein, a host cell receptor, sialylated glycans such as SAs and SA-containing gangliosides, cell adhesion molecules (CAMs), such as igSF members including CD4, JAM-A, CAR, integrins, such as αvβ3, PtdSer receptors, cell immunoglobulin and mucin domain (TIM), Tyro3, Axl, Mer (TAM), and ACE2 receptor. Preferred is the functionalized nano- or microparticle for use according to the present invention, wherein the virus-binding peptide is selected from LCB1, and AHB2.

Virus-binding peptides and minibinders can be successfully designed according to methods in the art (see, for example, Cao, L., Coventry, B., Goreshnik, I. et al. Design of protein-binding proteins from the target structure alone. Nature 605, 551-560 (2022). https://doi.org/10.1038/s41586-022-04654-9; [8]; [26]).

As mentioned above, preferred is the functionalized nano- or microparticle for use according to the present invention, wherein said binding between the virus-binding peptide and/or the virus-binding small molecule and the at least one target virus outcompetes binding of the target virus to its host cell receptor.

Therefore, the affinities between the virus-binding peptide and/or the virus-binding small molecule of the functionalized nano- or microparticle for use according to the present invention lie in nanomolar to picomolar range, preferably with dissociation constant (*K*_{d}) values in the range from below 100 nM, preferably between about 1 pM and 20 nM, more preferably between about 1pM and 100 pM.

In general, any suitable host cell receptor, i.e. the "entry point" of the viral infection in the mammalian subject, may be used for the design of the virus-binding peptide and/or the virus-binding small molecule. Preferably, the receptor is selected from cell adhesion molecules (CAMs) that function in cell-to-cell and cell-to-extracellular matrix interactions. The broad family of CAM includes selectins, cadherins, integrins, and IgSF members. The ubiquitous expression and multifactorial function of CAMs in ligand binding, endocytosis, and signaling provides a plethora of possibilities for viruses to engage CAMs. Viruses including HIV, measles virus, reovirus, rhinovirus, adenovirus, poliovirus, and coxsackievirus B (CVB) utilize IgSF members as receptors. In addition, integrins serve as receptors for reovirus, rotavirus, adenovirus, West Nile virus (WNV), human metapneuomovirus (hMPV), foot-and-mouth disease virus (FMDV), and herpes simplex virus (HSV), as well as human cytomegalovirus HCMV and human herpesvirus-8.

In another embodiment of the present invention, the host cell receptor is selected from IgSF members, which have emerged as receptors for a wide range of viruses including enveloped and non-enveloped viruses with either DNA or RNA genomes, including reovirus, adenovirus, coxsackievirus, rabies virus, measles virus, and HIV.

In another embodiment of the present invention, the host cell receptor is selected from integrins, which are receptors to mediate multiple functions such as viral entry and activation of signaling events. For example, the hMPV, an enveloped, negative-sense single-stranded RNA paramyxovirus and cause of respiratory infections particularly in children, utilizes integrins αvβ1 and α5β1 receptors to mediate infection through interactions with the viral fusion (F) protein.

In yet another embodiment of the present invention, said host cell receptor is selected from PtdSer receptors, including T-cell immunoglobulin and mucin domain (TIM) and TYROS, AXL, and the MERTK family of receptor tyrosine kinases (TAMs), which have been shown to serve as receptors for enveloped viruses from multiple virus families and viruses within the same viral family. PtdSer receptors have been reported to mediate viral entry of a number of enveloped viruses including the filoviruses EBOV and Marburgvirus (MARV); flaviviruses WNV, dengue virus (DENV), and Zika virus (ZIKV), and arenavirus Lassa Virus, and poxvirus vaccinia virus.

In yet another embodiment of the present invention, the host cell receptor is selected from SA receptors which mediate either binding to the host cell surface and/or entry. SV40, BKPyV, and PyV all utilize ganglioside receptors containing α2,3- and α2,8-linked SA to mediate viral attachment to respective host cells.

In one particularly preferred embodiment of the present invention, the at least one virus-binding peptide is selected from the angiotensin-converting enzyme 2 (ACE2), which is used by SARS-CoV-2 as the cellular entry receptor.

Obviously, the virus-binding peptide and/or the virus-binding small molecule must be designed to functionally also inhibit the binding of or to outcompete the binding of the target virus to its target receptor/protein on the mammalian cell as naturally targeted by the target virus.

In the context of the present invention, the virus-binding peptide may be suitably modified, e.g. in order to be immobilized onto the particle, any thus may comprises at least one terminal cysteine residue. The cysteine residue may optionally be modified to carry an azide group. In its azide form, the virus binder is no longer prone to dimerization and can be covalently bound to the nano- or microparticle surface. The virus binder azide can for example react with alkynes attached to a surface (e.g. of a nanoparticle core) in a strain-promoted azide-alkyne click reaction.

The present invention, in one preferred embodiment, furthermore uses the advantages of click-chemistry in order to quickly and conveniently allow for a modification of the combinations between particles, linkers and virus-binding particles. This multimodal set of possibilities allows for a relatively simple modification and exchange of the linkers and the virus-binding peptides. This enables a quick adaptation in cases of changes in the viruses for new variants of concern, as experiences in case of SARS-CoV-2 or to different viruses.

The person of skill is readily able to apply click-chemistry as exemplified by the cysteine and use of DCBO as above to other binding groups and pairs.

According to the invention, the immobilization of the virus-binding peptide or virus-binding small molecule to the nano- or microparticle surface may be direct or indirect, for example through conjugation, physical adsorption, linkers, and/or covalent linkage. In a preferred embodiment of the functionalized nano- or microparticle for use according to the present invention, the immobilization is a succinimidyl thioether bond or a bibenzocyclooctyne (DBCO) azide ligation. The peptide virus binders or virus-binding proteins may be attached or linked to the nano- or microparticle either in a covalent manner through chemical coupling strategies known to the skilled person or through physical adsorption.

In a preferred embodiment of the present invention, the peptide virus binders or virus-binding proteins is immobilized onto the surface of the nano- or microparticle through non-covalent interaction, particularly wherein the nano- or microparticle comprises PLGA.

In a preferred embodiment of the functionalized nano- or microparticle for use according to the present invention, the functionalized nano- or microparticle further comprises at least one antiviral drug. Preferably, the drug is included into the particle, or integrated into the membrane thereof, if the particle comprises a membrane. Antiviral drugs are, for example selected from at least one of neuraminidase inhibitors (e.g., oseltamivir, zanamivir), favipiravir, remdesivir, ribavirin (tribavirin), interferon alfa-2b/ribavirin systemic, interferon alpha 2a or 2b, inclusive any pegylated versions, chloroquine or hydroxychloroquine (given in combination with azithromycin), dolutegravir + rilpivirine (JULUCA^{®}), dolutegravir + lamivudine (DOVATO^{®}), ritonavir + lopinavir (Kaletra^{®}), bictegravir + tenofovir alafenamide + emtricitabine (BIKTARVY^{®}), dolutegravir + abacavir + lamivudine (TRIUMEQ^{®}), elvitegravir + cobicistat +emtricitabine + tenofovir alafenamide (GENVOYA^{®}), and elvitegravir + cobicistat + emtricitabine + tenofovir disoproxil fumarate + emtricitabine (STRIBILD^{®}).

For diagnostic purposes, the functionalized nano- or microparticle for use according to the present invention may further comprise at least one detectable label or marker, such as an enzyme, antigen, weight and/or fluorescent marker.

In a multicellular organism's immune system, phagocytosis as a type of endocytosis is a major mechanism used to remove pathogens and cell debris. The ingested material is then digested in the phagosome. Bacteria, dead tissue cells, and small mineral particles are all examples of objects that may be phagocytized. The present invention provides a method to harness the phagocytosis pathway for viral clearance. This provides an advantage when compared to methods of the state of the art, where nanoparticles comprising viral components are made merely in order to trigger a directed immune response against a certain target virus.

Innate immune cells like macrophages, monocytes, neutrophilic granulocytes and Dendritic cells and also epithelial and endothelial cells engulf and take-up pathogens or particles by a process called phagocytosis in order to eliminate these. Although these cells do play a role in the immunopathobiology of viral infection, many virions are not susceptible to phagocytosis due to their small size. In the present invention, it is made possible to harness the phagocytosis process for viral clearance, which will effectively neutralize and remove virus before they can interact with their respective host cell.

According to the invention, the aggregation is driven by the high affinity binding between the virus binder on the surface of the nano- or microparticle and the virions. One such functionalized nano- or microparticle may sequester multiple virions. Each target virus can interact with the nanoparticle through multiple binding events, thus potentiating high binding avidity and reducing the probability of complex dissociation. Additionally, the diameter of the particle-virus-complex is large enough to be phagocytosed by the above-mentioned cells allowing the digestion of the virus in a mature phagolysosome.

Yet another aspect of the present invention thus relates to an aggregate comprising at least one functionalized nano- or microparticle for use according to the present invention and at least one target virus for use in the prevention and/or treatment of target virus infection in a mammalian subject, wherein said prevention and/or treatment comprises phagocytosis, such as macrophage-mediated phagocytosis in said mammalian subject.

Preferred is the functionalized nano- or microparticle for use according to the present invention or the aggregate for use according to the present invention, wherein said at least one target virus is selected from the group of HIV, measles virus, reovirus, rhinovirus, adenovirus, poliovirus, coxsackievirus B, reovirus, rotavirus, adenovirus, West Nile virus, human metapneuomovirus (hMPV), foot-and-mouth disease virus (FMDV), herpes simplex virus (HSV), HPV, human cytomegalovirus HCMV, human herpesvirus-8, rabies virus, hMPV, paramyxovirus, filoviruses, Ebola virus (EBOV), Marburgvirus (MARV), flaviviruses, dengue virus (DENV), Zika virus (ZIKV), Lassa Virus, poxvirus, SV40, BKPyV, PyV, Influenza A virus (IAV), Orthomyxoviridae, MERS-CoV, betacoronavirus, SARS-S, and SARS-CoV-2.

Another aspect of the present invention then relates to a pharmaceutical composition comprising the functionalized nano- or microparticle for use according to the present invention or the aggregate for use according to the present invention for use in the prevention and/or treatment of target virus infection in a mammalian subject, wherein said pharmaceutical composition additionally comprises suitable excipients and/or auxiliary agents.

Preferred is the pharmaceutical composition for according to the present invention, wherein said composition is formulated for intravenous injection, a drinking solution/suspension, a solution/suspension suitable for aerosol production and inhalation, or dry powder for inhalation.

The pharmaceutical composition may be formulated to be administered by intravenous injection, drinking a solution/suspension, or by inhalation. The pharmaceutical composition thus can be a tablet, capsule, granule, powder, sachet, reconstitutable powder, dry powder inhaler and/or chewable. Such solid formulations may additionally comprise excipients and other ingredients in suitable amounts, such as cellulose, cellulose microcrystalline, polyvidone, in particular FB polyvidone and/or magnesium stearate.

Preferred is the pharmaceutical composition according to the present invention formulated for inhalation, wherein said functionalized nano- or microparticle size is between about 400 to 700 nm for diffusion through the lung mucus layer. In this case, the dosage as applied can preferably be reduced because of the application of the drug directly to the site of action, *i.e.* the respiratory tract.

The pharmaceutical compositions according to the present invention may optionally comprise a pharmaceutically acceptable carrier. Pharmaceutically acceptable carriers or excipients include diluents (fillers, bulking agents, e.g. lactose, microcrystalline cellulose), disintegrants (e.g. sodium starch glycolate, croscarmellose sodium), binders (e.g. PVP, HPMC), lubricants (e.g. magnesium stearate), glidants (e.g. colloidal SiO₂), solvents/co-solvents (e.g. aqueous vehicle, Propylene glycol, glycerol), buffering agents (e.g. citrate, gluconates, lactates), preservatives (e.g. Na benzoate, parabens (Me, Pr and Bu), BKC), anti-oxidants (e.g. BHT, BHA, Ascorbic acid), wetting agents (e.g. polysorbates, sorbitan esters), thickening agents (e.g. methylcellulose or hydroxyethylcellulose), sweetening agents (e.g. sorbitol, saccharin, aspartame, acesulfame), flavoring agents (e.g. peppermint, lemon oils, butterscotch, etc.), humectants (e.g. propylene, glycol, glycerol, sorbitol). Other suitable pharmaceutically acceptable excipients are inter alia described in Remington's Pharmaceutical Sciences, 15th Ed., Mack Publishing Co., New Jersey (1991) and Bauer et al., Pharmazeutische Technologic, 5th Ed., Govi-Verlag Frankfurt (1997). The person skilled in the art knows suitable formulations for cilengitide and/or respective derivatives and will readily be able to choose suitable pharmaceutically acceptable carriers or excipients, depending, *e.g.,* on the formulation and administration route of the pharmaceutical composition.

It is to be understood that the pharmaceutical composition according to the present invention is for use in mammalian subjects. The pharmaceutical composition of the present invention may be used individually or in co-therapy approaches, *i.e.,* in co-administration with other medicaments or drugs and/or any other therapeutic agent which might be beneficial in the context of the methods of the present invention.

In a further aspect, the above object is solved by providing a pharmaceutical composition comprising a functionalized nano- or microparticle according to the present invention for the prevention or treatment of viral infection, wherein the pharmaceutical composition optionally further comprises suitable excipients and/or auxiliary agents, as described herein.

In a further aspect, the above object is solved by providing a method for detecting a target virus and/or target virus specific anti-virus antibodies in a biological sample obtained from a mammalian subject, comprising contacting the functionalized nano- or microparticle for use according to the present invention with said biological sample, and detecting binding of said target virus and/or target virus specific anti-virus antibodies to said particle and/or detecting forming of aggregates of said target virus with said functionalized nano- or microparticle, wherein a binding and/or forming of aggregates is indicative for the target virus and/or target virus specific anti-virus antibodies in the biological sample. Methods and strategies to perform this method can be taken from the respective literature, and may be readily modified for the specific detection.

In a related embodiment according to the present invention, the functionalized nano- or microparticle further comprises at least one detectable label or marker, for example, a fluorescent tag, so that the aggregates formed between the functionalized nano- or microparticle and target virus can be detected.

Preferred is the method according to the present invention, further comprising the step of quantifying the amount of a target virus and/or target virus specific anti-virus antibodies in the biological sample obtained from the mammalian subject based on the binding and/or forming of aggregates in the biological sample. Here, the number of viral particles bound onto the functionalized nano- or microparticle can be determined. The skilled person is aware of different techniques in detecting or quantifying the labeled particles. For example, a recombinant wt-SARS-CoV-2 RBD fluorescently labeled with the Alexa Fluor 488 dye (RBDAF488), that is incubated the functionalized nano- or microparticle with LCB 1 minibinder, can be detected using flow cytometry.

In the context of the present invention, any suitable sample such as a biological sample can be used for the assay of the present invention, for example the sample is a blood or serum sample derived from the mammal, or a pooled sample from a group of mammalian subjects.

The object of the present invention is furthermore solved by a diagnostic composition for detecting a target virus and/or specific anti-virus antibodies in a sample obtained from a mammalian subject, comprising the step of contacting the functionalized nano- or microparticle according to the present invention with said biological sample, and detecting of binding of said virus and/or said specific antibodies to said particle, thereby detecting said target virus and/or said antibodies in said biological sample.

In this aspect of the present invention, the nano- or microparticle is used as a diagnostic tool, where the target virus particles or antibodies as bound to the nano- or microparticle are detected as part of a diagnosis. Preferred is the diagnostic composition according to the present invention, wherein said functionalized nano- or microparticle comprises a detectable label or marker as described above.

Yet another aspect of the present invention then relates to a kit, such as a diagnostic kit, for performing the method according to the present invention, comprising at least one functionalized nano- or microparticle for use according to the present invention, or the above diagnostic composition, together with suitable auxiliary agents. The kit may comprise the material for performing a method according to the present invention in one or separate containers, optionally together with auxiliary agents and/or instructions for performing said method.

Preferred is the use of said diagnostic kit according to the present invention for the detection of target viruses and/or anti-virus antibodies in a biological sample obtained from a mammalian subject, in particular in the context of betacoronaviral infection, such as COVID-19. Further preferred is the use of the kit according to the present invention for detecting or quantifying a target virus and/or target virus specific anti-virus antibodies in a biological sample obtained from a mammalian subject.

Yet another aspect of the present invention then relates to a method for preventing and/or treating a target virus infection in a mammalian subject, comprising administering the functionalized nano- or microparticle for use according to the present invention, the aggregate for use according to the present invention, or the pharmaceutical composition for use according to the present invention to a mammalian subject in need of preventing and/or treating a target virus infection.

Preferred is the method according to the present invention, wherein the target virus is selected from the group of HIV, measles virus, reovirus, rhinovirus, adenovirus, poliovirus, coxsackievirus B, reovirus, rotavirus, adenovirus, West Nile virus, human metapneuomovirus (hMPV), foot-and-mouth disease virus (FMDV), herpes simplex virus (HSV), HPV, human cytomegalovirus HCMV, human herpesvirus-8, rabies virus, hMPV, paramyxovirus, filoviruses, Ebola virus (EBOV), Marburgvirus (MARV), flaviviruses, dengue virus (DENV), Zika virus (ZIKV); Lassa Virus, poxvirus, SV40, BKPyV, PyV, Influenza A virus (IAV), Orthomyxoviridae, MERS-CoV, betacoronavirus, SARS-S, and SARS-CoV-2.

By "treatment" or "treating" is meant any treatment of a disease or disorder, in a subject, such as a mammal or human, including: preventing or protecting against the disease or disorder, that is, causing, the clinical symptoms of the disease not to develop; inhibiting the disease, that is, arresting or suppressing the development of clinical symptoms; and/or relieving the disease, that is, causing the regression of clinical symptoms. By "amelioration" is meant the prevention, reduction or palliation of a state, or improvement of the state of a subject; the amelioration of a stress is the counteracting of the negative aspects of a stress. Amelioration includes, but does not require, complete recovery or complete prevention of a stress.

Taken together, the combined competition with host cell receptor and binding of virions to the functionalized nano- or microparticles to form aggregates that can be phagocytosed will reduce the viral load in the host and thus lead to a quick recovery of the mammalian subject suffering from a viral infection, particularly from COVID-19.

According to the present invention a mammalian subject may be selected from a rodent, cattle, pets, and a human. The subject may be a patient.

In the context of the present invention, the term "target virus" refers to a virus in its viral particle or virion form.

The methods according to the present may be performed in vivo or in vitro.

In a further aspect, the present invention provides a method for producing a functionalized nano- or microparticle according to the present invention, the method comprising the steps of suitably providing a nano- or microparticle as described herein, wherein the functionalized nano- or microparticle has an overall diameter of at least about 400 nm, preferably between about 400 to 2000 nm, and more preferably between about 600 and 700 nm, suitably providing at least one virus-binding peptide and/or the virus-binding small molecule that binds to the at least one target virus with nano- to picomolar affinity as described herein, and suitably immobilizing the at least one virus-binding peptide and/or the virus-binding small molecule onto the surface of the nano- or microparticle.

In a further aspect, the present invention provides a method for producing an aggregate comprising at least one functionalized nano- or microparticle for use according to the present invention comprising the steps of producing a nano- or microparticle as described herein, and binding the functionalized nano- or microparticle for use to at least one target virus to form the aggregate. The aggregate is suitable to be phagocytosed, i.e. is larger than 400 nm, for example between about 400 and 5000 µm.

In another embodiment of the method according to the present invention, the material for forming the nano- or microparticle according to the invention may be additionally primed with a suitable functional group for conjugation with the high affinity the at least one virus-binding peptide and/or the virus-binding small molecule in a subsequent immobilization step. This functional group may particularly be one that is used in Click chemistry reaction, particularly a maleimide group or a dibenzylcyclooctyne (DBCO) group.

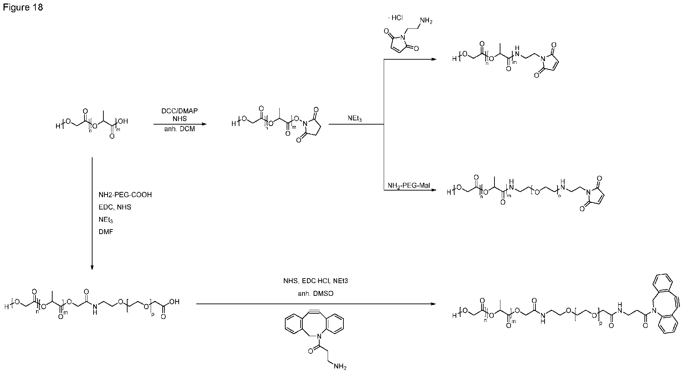

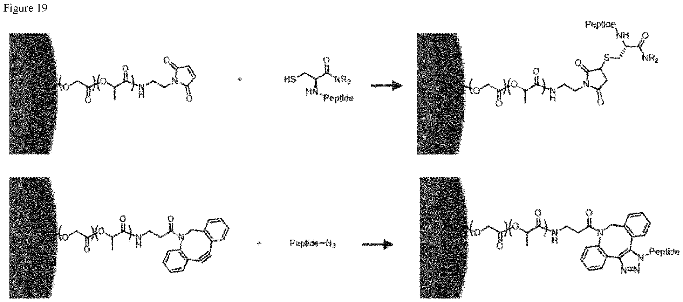

As examples, these functional groups may be added to the polymer through suitable chemical treatment known in the state of the art (see also Figures 18 and 19). For attaching the maleimide group, for example, the polymer material comprising the nano- or microparticle undergoes ester activation using N-hydroxysuccinimide (NHS), and is subsequently amidated using maleimide- or PEG-maleimide amine. Alternatively, for attaching the DBCO group, as another example, the polymer material undergoes firstly an amidation with NH₂-PEG-COOH via NHS ester activation and subsequently, the DBCO group is attached in an NHS ester activation reaction with DBCO amine.

The person skilled in the art will be aware of various methods for forming nano- or microparticles. In a preferred embodiment of the method according to the invention, the nano- and or microparticle is formed using a single emulsion technique. For example, the material forming the particle may be dissolved in ethyl acetate forming an organic solution. Said organic solution can be mixed with an aqueous phase containing poly(vinyl alcohol) (PVA). When the organic solvent is evaporated, the nano- or microparticle is formed and can be purified by ultrafiltrati on.

Another embodiment of the present invention then relates to a method to produce the at least one virus-binding peptide using *Escherichia coli,* comprising expressing a recombinant plasmid coding for the desired virus-binding peptide in a suitable *E.coli* strain. The strain could be cultured to over-express the virus-binding peptide, and/or to excrete the peptide into the medium. The peptide can then be isolated from the cells or the medium using conventional techniques.

In another related embodiment of the method according to the present invention, the virus-binding peptides may be expressed with a suitable tag for purification, for example, an N-terminal His6-tag. In a specific example, the virus binder with the His6-tag can be purified using Co-chelate chromatography, and in a subsequent step, the tag can be removed by a tobacco etch virus (TEV) protease digestion, followed by size exclusion chromatography.

In yet another embodiment of the method according to the present invention, the virus-binding peptide as produced carry at least one terminal cysteine residue. For example, the virus-binding peptide produced could be flanked by short sequences of Ser and Gly which carry Cys residues at the C-terminus. This terminal cysteine residue can be used for covalent attachment to the nano- or microparticle in a subsequent step.

In another preferred embodiment of the method according to the present invention, the binding affinity of the virus-binding peptide and the target viral particle can be verified to, for example, optimize the virus binder composition. Techniques such as mutagenesis and surface plasmon resonance (SPR) spectroscopy can be used for this purpose.

In the context of the present invention, the term "about" shall mean a deviation of +/- 10% of the given value, unless indicated otherwise.

The object of the present invention was to establish a new therapeutic approach to bind, neutralize and clear in particular SARS-CoV-2 virions using nanoparticles. These particles are decorated with a high-affinity minibinder which outcompetes the natural SARS-CoV-2·hACE2 interaction by binding the SARS-CoV-2 spike RBD with a higher specificity and higher affinity than hACE2. Further, the inventors showed that by complexation of several virions to nanoparticles increasing the size of virions that is required that they are degraded *via* phagocytosis and intracellular processing by host cells.

The inventors showed that nanoparticles equipped with covalently bound minibinder, like the compounds P, 28A and 29A, were able to bind VSVΔG-S virions and channel them to phagocytosis and intracellular degradation *in vitro.* The inventors thus identified a new way to achieve viral clearance with potential applicability to treat all kind of viral infections in humans. In principle, this approach is not only applicable to SARS-CoV-2, but also to other airborne viruses small enough to escape phagocytosis like influenza, provided miniproteins binding the virus with sufficient affinity are available to attach them on the particle surface.

Apart from facilitating phagocytosis, the nanoparticles equipped with covalently bound minibinder are able to block the virus and neutralize it as shown in the SARS-CoV-2 neutralization assays. In these assays, not only those VBKs that displayed covalently bound minibinder on their surface revealed neutralizing activity, but also those particles that merely adsorbed the minibinder. This indicates that the nanoparticles may also serve as delivery vehicles for the free minibinder, which has already been successfully applied to animal models of SARS-CoV-2 infection [28].

In addition to their remarkable virus-neutralizing activity *in vitro,* the VBKs did not affect the cell viability, nor did they permeate the cell membrane.

The present invention relates to the following items.

Item 1. A functionalized nano- or microparticle for use in the prevention and/or treatment of target virus infection in a mammalian subject, comprising, a) at least one nano- or microparticle, and b) at least one virus-binding peptide and/or at least one virus-binding small molecule immobilized onto the surface of the nano- or microparticle, wherein the virus-binding peptide and/or the virus-binding small molecule binds to the at least one target virus with nano- to picomolar affinity, and wherein the functionalized nano- or microparticle has an overall diameter of at least about 400 nm, preferably between about 400 to 2000 nm, and more preferably between about 600 and 700 nm.

Item 2. The functionalized nano- or microparticle for use according to Item 1, wherein said binding between the virus-binding peptide and/or a virus-binding small molecule and the at least one target virus outcompetes binding of the target virus to its host cell receptor.

Item 3. The functionalized nano- or microparticle for use according to Item 1 or 2, wherein said functionalized nano- or microparticle is substantially spherical in shape.

Item 4. The functionalized nano- or microparticle for use according to any one of Items 1 to 3, wherein said nano- or microparticle comprises a suitable organic or inorganic material, such as, for example, a metal, a plastic, a polymer, such as, for example, PLA, PLGA, PLGH, polyglutamate, styrene-maleic acid copolymer or PE, or is a nanosphere composed of membranes and/or lipids, such as a liposome, preferably wherein said nano- or microparticle comprises PLGA-Mal, PLGA-PEG-Mal, PLGA-DY550, PLGA-PEG, PLGA-PEG-DBCO, and/or combinations thereof.

Item 5. The functionalized nano- or microparticle for use according to any one of Items 1 to 4, wherein the virus-binding peptide is selected from a minibinder protein, a host cell receptor, sialylated glycans such as SAs and SA-containing gangliosides, cell adhesion molecules (CAMs), such as igSF members including CD4, JAM-A, CAR, integrins, such as αvβ3, PtdSer receptors, cell immunoglobulin and mucin domain (TIM), Tyro3, Axl, Mer (TAM), and ACE2 receptor.

Item 6. The functionalized nano- or microparticle for use according to Item 5, wherein the virus-binding peptide is selected from LCB1 and AHB2.

Item 7. The functionalized nano- or microparticle for use according to Item 5 or 6, wherein said virus-binding peptide comprises at least one terminal cysteine residue, said cysteine residue is optionally modified to carry an azide group.

Item 8. The functionalized nano- or microparticle for use according to any one of Items 1 to 7, wherein said immobilization of the virus-binding peptide or virus-binding small molecule to the nano- or microparticle surface is direct or indirect, for example through conjugation, physical adsorption and/or covalent linkage.

Item 9. The functionalized nano- or microparticle for use according to Item 8, wherein the immobilization is a succinimidyl thioether bond or a bibenzocyclooctyne (DBCO) azide ligation.

Item 10. The functionalized nano- or microparticle for use according to any one of Items 1 to 9, wherein said functionalized nano- or microparticle further comprises at least one antiviral drug.

Item 11. The functionalized nano- or microparticle for use according to any one of Items 1 to 10, wherein said functionalized nano- or microparticle further comprises at least one detectable label or marker.

Item 12. An aggregate comprising at least one functionalized nano- or microparticle for use according to any one of Items 1 to 11 and at least one target virus for use in the prevention and/or treatment of target virus infection in a mammalian subject, wherein said prevention and/or treatment comprises phagocytosis, in particular macrophage mediated phagocytosis in said mammalian subject.

Item 13. The functionalized nano- or microparticle for use according to any one of Items 1 to 11 or the aggregate for use according to Item 12, wherein said at least one target virus is selected from the group of HIV, measles virus, reovirus, rhinovirus, adenovirus, poliovirus, coxsackievirus B, reovirus, rotavirus, adenovirus, West Nile virus, human metapneuomovirus (hMPV), foot-and-mouth disease virus (FMDV), herpes simplex virus (HSV), HPV, human cytomegalovirus HCMV, human herpesvirus-8, rabies virus, hMPV, paramyxovirus, filoviruses, Ebola virus (EBOV), Marburgvirus (MARV), flaviviruses, dengue virus (DENV), Zika virus (ZIKV), Lassa Virus, poxvirus, SV40, BKPyV, PyV, Influenza A virus (IAV), Orthomyxoviridae, MERS-CoV, betacoronavirus, SARS-S, and SARS-CoV-2.

Item 14. A pharmaceutical composition comprising the functionalized nano- or microparticle for use according to any one of Items 1 to 11 or 13 or the aggregate for use according to Item 12 or 13 for use in the prevention and/or treatment of target virus infection in a mammalian subject, wherein said pharmaceutical composition additionally comprises suitable excipients and/or auxiliary agents.

Item 15. The pharmaceutical composition for according to Item 14, wherein said composition is formulated for intravenous injection, a drinking solution/suspension, a solution/suspension suitable for aerosol production and inhalation, or dry powder for inhalation.

Item 16. The pharmaceutical composition for use according to Item 14 or 15, wherein said functionalized nano- or microparticle has size between about 400 to 700 nm and is optionally suitable for administration through inhalation.

Item 17. A method for detecting a target virus and/or target virus specific anti-virus antibodies in a biological sample obtained from a mammalian subject, comprising contacting the functionalized nano- or microparticle for use according to any one of Items 1 to 11 with said biological sample, and detecting binding of said target virus and/or target virus specific anti-virus antibodies to said particle and/or detecting forming of aggregates of said target virus with said functionalized nano- or microparticle, wherein a binding and/or forming of aggregates is indicative for the target virus and/or target virus specific anti-virus antibodies in the biological sample.

Item 18. The method according to Item 17, wherein said functionalized nano- or microparticle further comprises at least one detectable label or marker.

Item 19. The method according to Item 17 or 18, wherein the sample is a blood or serum sample derived from the mammal, or a pooled sample from a group of subjects.

Item 20. The method according to any one of Items 17 to 19, further comprising the step of quantifying the amount of a target virus and/or target virus specific anti-virus antibodies in the biological sample obtained from the mammalian subject based on the binding and/or forming of aggregates in the biological sample.

Item 21. A kit for performing the method according to any one of Items 17 to 20, comprising at least one functionalized nano- or microparticle for use according to any one of the Items 1 to 11, together with suitable auxiliary agents.

Item 22. Use of the kit according to Item 21 for detecting or quantifying a target virus and/or target virus specific anti-virus antibodies in a biological sample obtained from a mammalian subj ect.

Item 23. A method for preventing and/or treating a target virus infection in a mammalian subject, comprising administering the functionalized nano- or microparticle for use according to any one of Items 1 to 11 or 13, the aggregate for use according to Item 12 or 13, or the pharmaceutical composition for use according to any one of Items 14 to 16 to a mammalian subject in need of preventing and/or treating a target virus infection.

Item 24. The method according to Item 23, wherein the target virus is selected from the group of HIV, measles virus, reovirus, rhinovirus, adenovirus, poliovirus, coxsackievirus B, reovirus, rotavirus, adenovirus, West Nile virus, human metapneuomovirus (hMPV), foot-and-mouth disease virus (FMDV), herpes simplex virus (HSV), HPV, human cytomegalovirus HCMV, human herpesvirus-8, rabies virus, hMPV, paramyxovirus, filoviruses, Ebola virus (EBOV), Marburgvirus (MARV), flaviviruses, dengue virus (DENV), Zika virus (ZIKV); Lassa Virus, poxvirus, SV40, BKPyV, PyV, Influenza A virus (IAV), Orthomyxoviridae, MERS-CoV, betacoronavirus, SARS-S, and SARS-CoV-2.

The present invention will now be described further in the following examples with reference to the accompanying Figures and sequence listing, nevertheless, without being limited thereto. For the purposes of the present invention, all references as cited herein are incorporated by reference in their entireties.

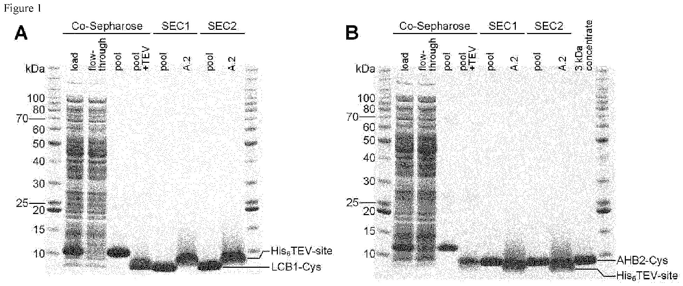

Figure 1 shows the purification of the recombinant minibinders LCB1 (A) and AHB2 (B). SDS-PAGE analysis of fractions from sequential cobalt chelate affinity chromatography, TEV protease treatment and size exclusion chromatography (SEC).

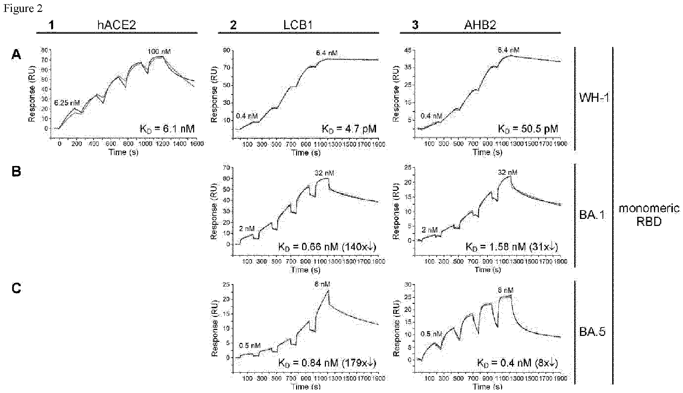

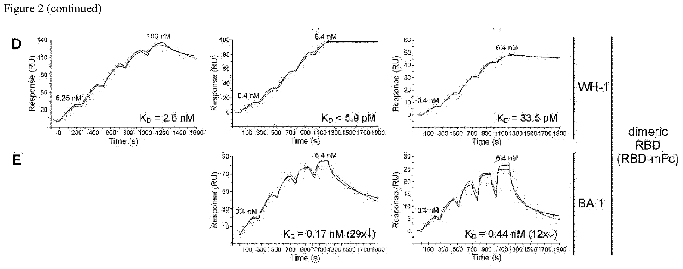

Figure 2 shows the SPR-based binding analysis of monomeric RBDs and dimeric RBD-mFc fusion constructs to hACE2, LCB1 and AHB2. Interactions with Wuhan-like variant (A and D) and omicron SARS-CoV-2 RBD variants BA.1 and BA.5 (B, C and E) were measured. Sensorgrams for RBD and RBD-mFc binding to hACE2 (column 1), LCB1 (column 2) and AHB2 (column 3) are shown. Kinetic titration series binding responses of the indicated RBD and RBD-mFc concentrations (black lines) are overlaid with the best fit derived from a 1:1 interaction model, including a mass transport term (gray lines). Dissociation constant (K_{D}) values are displayed inside the graphs. The reduction in affinity compared to the wt-RBD or dimeric wt-RBD is given in parentheses.

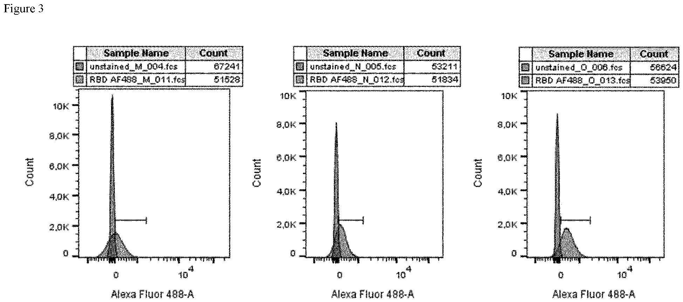

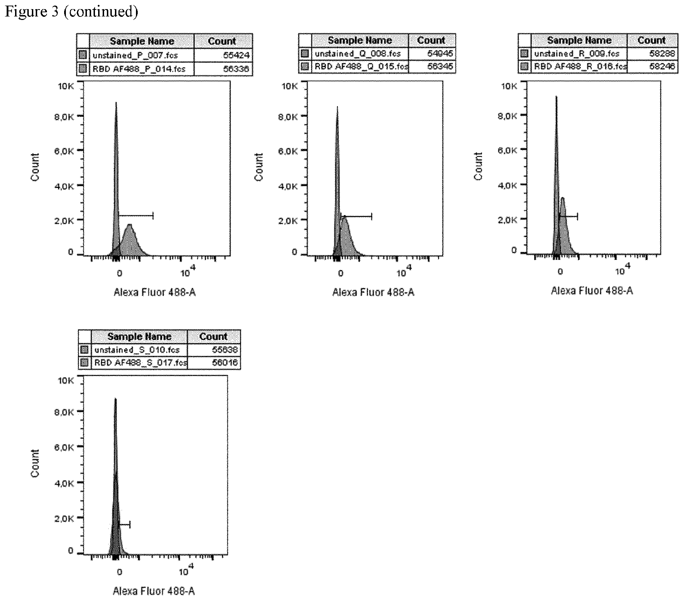

Figure 3 shows the flow cytometry analysis of particles M-S with (dark gray) and without (gray) preincubation with a fluorescently labeled RBD.

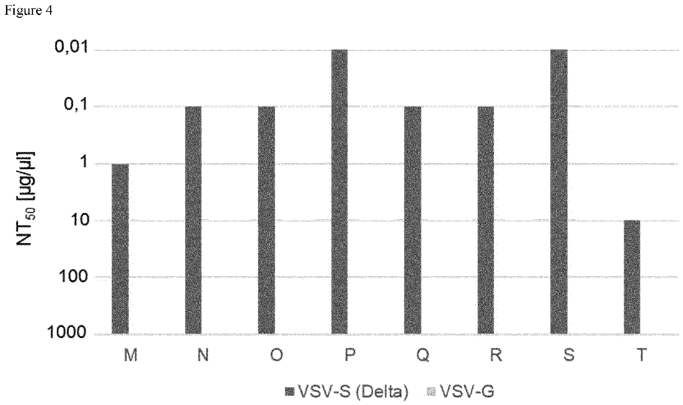

Figure 4 shows the neutralizing titers of VBKs M-T. VBKs M-T were incubated with VSVΔG particles pseudotyped with the SARS-CoV-2 spike surface protein (VSVΔG-S) for 1 h at 37 °C and subsequently added onto Vero cells. 16 h post infection, GFP positive cells were counted.

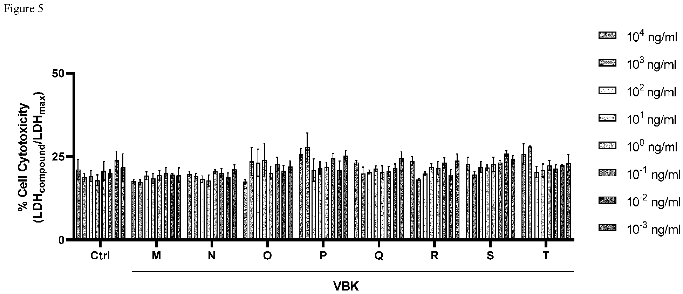

Figure 5 shows an LDH release assay after 48 h of co-incubation of Calu-3 cells with VBKs. Calu-3 cells were treated with the VBKs at eight different concentrations, and 48h post treatment the release of LDH, which represents cell membrane damage, was measured using a manufactured LDH release assay (Promega). Non-treated cells were used as negative control (Ctrl). Cell cytotoxicity (%) is shown as average ± SD from one biological replicate, performed in technical triplicates.

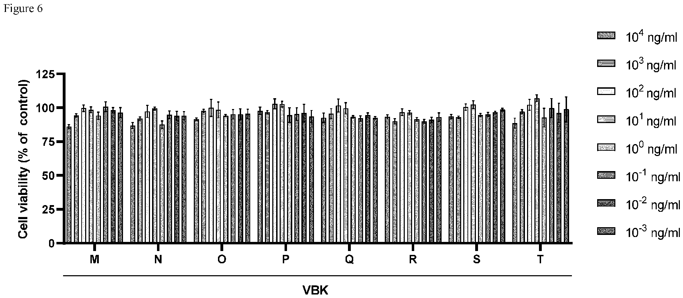

Figure 6 shows an MTT assay after 48 h of co-incubation of Calu-3 cells with VBKs. Calu-3 cells were treated with the VBKs at eight different concentrations and 48h post treatment the metabolic activity of the cells was measured using a manufactured cell proliferation assay (Promega). Non-treated cells were used as negative control. Cell viability (% of control) is shown as average ± SD from one biological replicate, performed in technical triplicates.

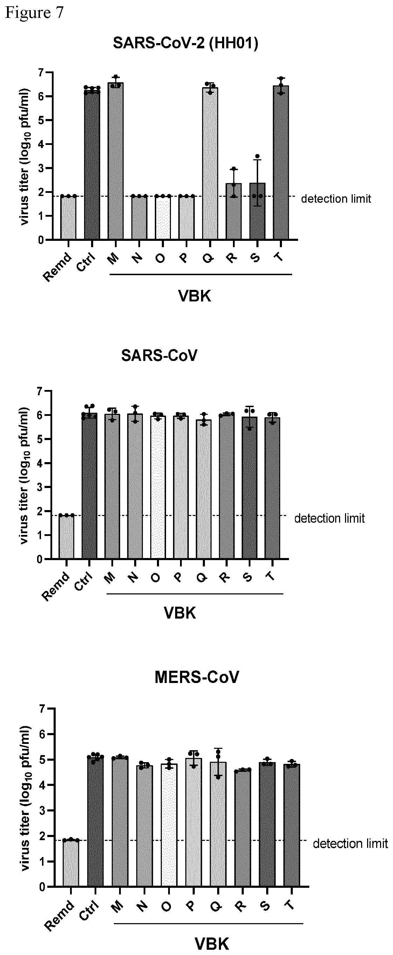

Figure 7 shows inhibition of SARS-CoV-2, SARS-CoV, and MERS-CoV replication by VBKs. Calu-3 cells were infected with the respective virus strain at an MOI of 0.01 and therapeutically treated with the VBKs at a single-dose of 10 µg/ml. Non-treated cells were used as negative control (Ctrl). Remdesivir (Remd, 10µM), a pan-CoV inhibitor, was used as positive control. At 48h post infection, infectious virus titers were determined in the cell culture supernatants by plaque test. Viral titers are shown as average log10 plaque-forming units/ml ± SD from one biological replicate, performed in technical triplicates. The detection limit is indicated with a dashed line.

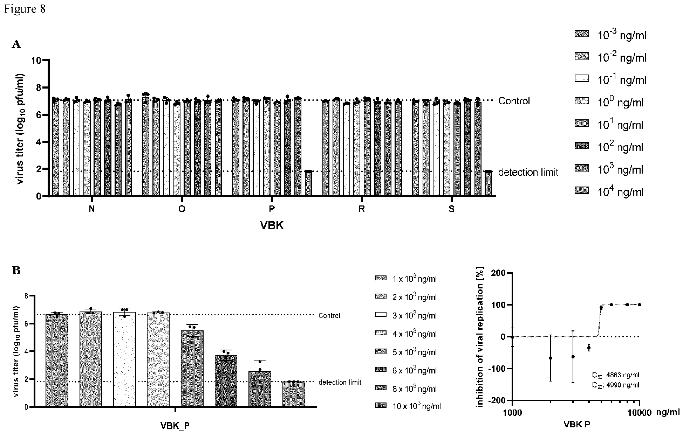

Figure 8 shows inhibition of SARS-CoV-2 replication by VBKs. Calu-3 cells were infected with SARS-CoV-2 HH01 at an MOI of 0.01 and therapeutically treated with the VBKs at eight different concentrations. Non-treated cells were used as negative control (Control). At 48h post infection, infectious virus titers were determined in the cell culture supernatants by plaque test. **A)** Viral titers are shown as average log10 plaque-forming units/ml ± SD from one biological replicate, performed in technical triplicates. **B)** For VBK_P, the IC₅₀ and IC₉₀ values were determined by non-linear regression using an adjusted concentration range from 1 to 10 µg/ml. Data are shown as average ± SD of inhibition of viral replication (% of control). The detection limit is indicated with a dashed line.

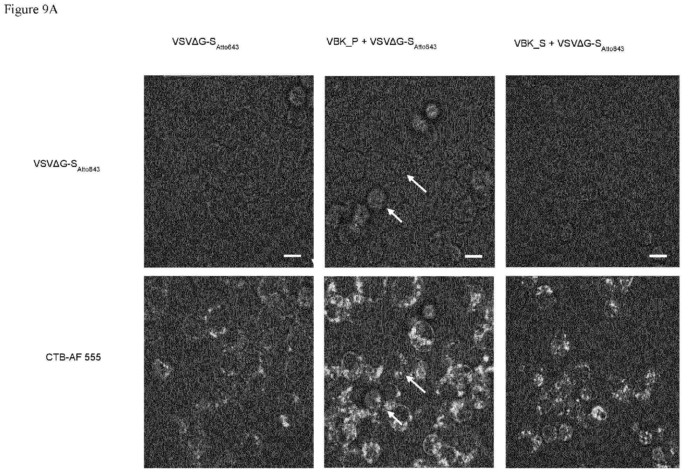

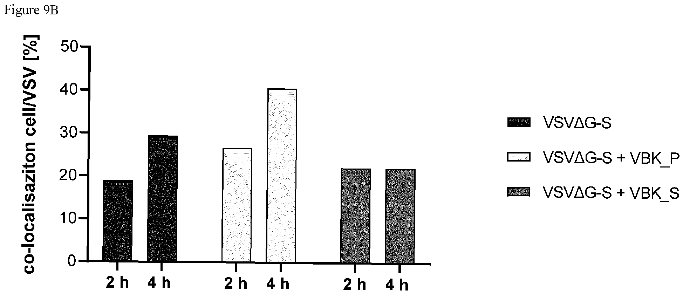

Figure 9 shows the evaluation of the phagocytosis ability of virus-nanoparticle complexes. **A)** CLSM micrographs merged with bright field images of RAW264.7 cells incubated with VSVΔG-S_{Atto643} virions or co-incubated with VSVΔG-S_{Atto643} virions and VBK_P or VBK_S. VSVΔG-S_{Atto643} virions appear as red puncta. Membranes are stained by the sphingolipid GM1 with CTB-Alexa Fluor 555 and appear green. VSVΔG-S_{Atto643} virions trapped in intracellular vesicles, like phagosomes, of macrophages are marked by arrows and appear as yellow overlay-signal of both used stainings. Scale 10µm **B)** Quantification of the virion uptake by signal co-localization.

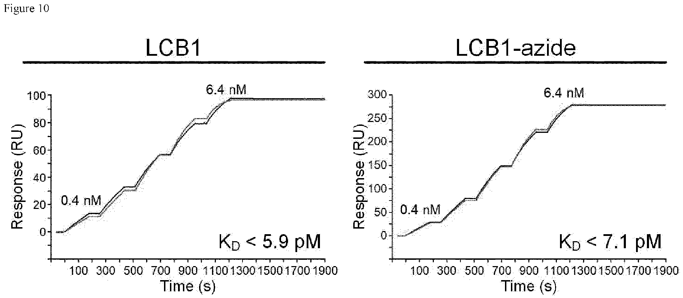

Figure 10 shows the comparison of SPR analyses of RBD-mFc (SARS-CoV-2 Wuhan-like variant) binding to sensor-immobilized LCB1 and LCB1-azide. Data are presented as described in the legend to Figure 1. The affinity was found to be too high to be determined accurately by SPR spectroscopy because the off-rate is outside the instrument limits. Therefore, the difference in affinity for RBD-mFc is not significant.

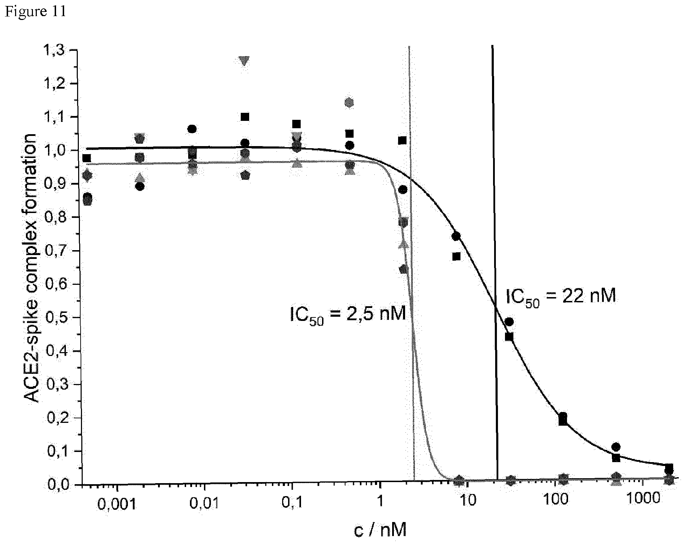

Figure 11 shows the competition of varying concentrations of LCB 1-azide with ACE2 (7.5 nM) for binding the trimeric spike of the wt-variant (blue) and of the omicron BA.1 variant (black). Regarding the wt-spike, the assay was performed with two different batches of LCB1-azide, each batch was assessed in duplicate (light gray triangles, dark gray pentagons and hexagons). The assay involving the omicron BA.1 spike was performed in duplicate (black). Red symbols represent outliers that were excluded from the fitting procedure.

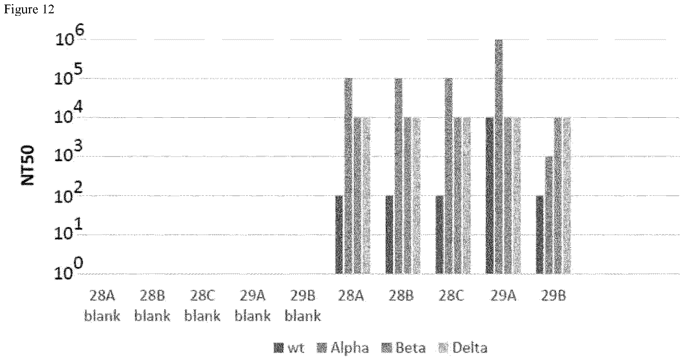

Figure 12 shows the half-maximal inhibitory concentration (IC₅₀) of different VBKs with respect to VSVΔG virions equipped with spike proteins of different SARS-CoV-2 variants.

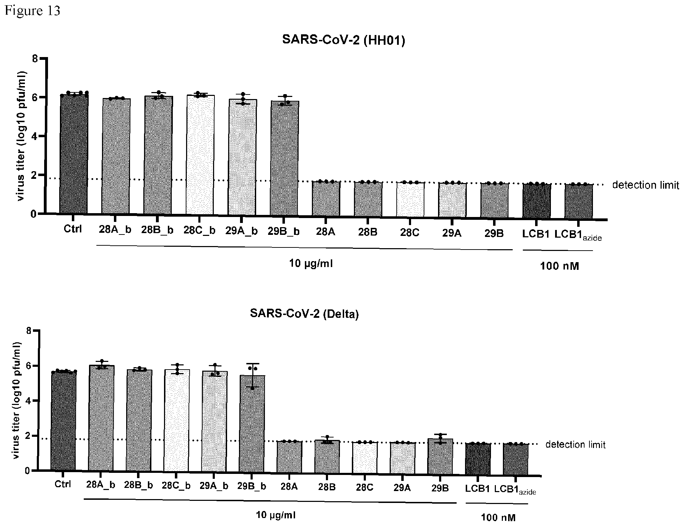

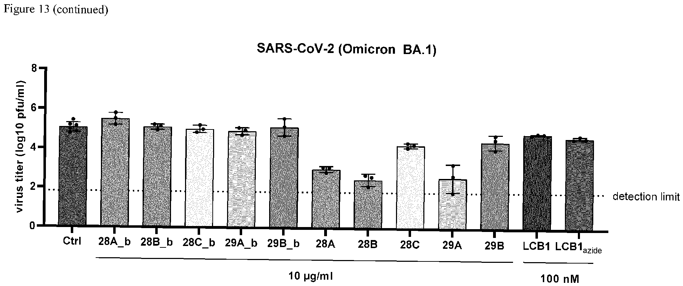

Figure 13 shows the antiviral activity of VBKs 28A-C and 29A-B (including their respective blank controls) against SARS-CoV-2 HH01 (Wuhan-like), Delta and Omicron BA.1 variants. Human Calu-3 cells were infected with the respective virus strain at an MOI of 0.01 and therapeutically treated with the VBKs at a single-dose of 10 µg/ml. Non-treated cells were used as negative control (Ctrl). Purified minibinders LCB1 and LCB1_{azide} (100 nM) were used as positive controls. At 48 h post infection, infectious virus titers were determined in the cell culture supernatants by plaque test. Viral titers are shown as average log10 plaque-forming units/ml ± SD from one biological replicate, performed in technical triplicates. The detection limit is indicated with a dashed line.

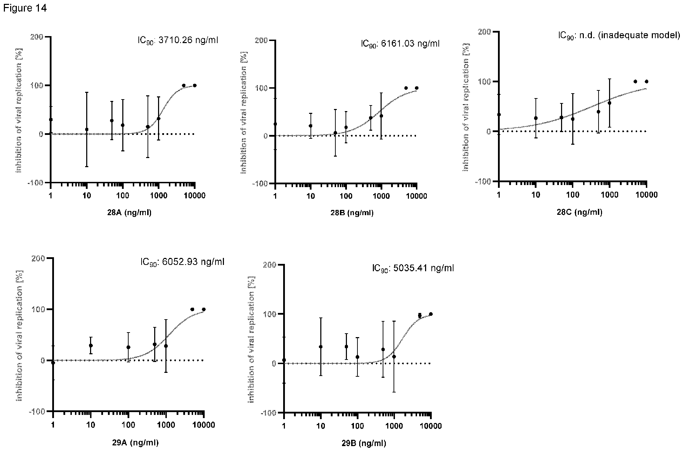

Figure 14 shows the determination of the IC₉₀ of VBKs 28A-C and 29A-B in a SARS-CoV-2 neutralization assay. Calu-3 cells were infected with SARS-CoV-2 HH01 at an MOI of 0.01 and therapeutically treated with the VBKs at eight different concentrations (range 0.001 to 10 µg/ml). Non-treated cells were used as negative control. At 48h post infection, infectious virus titers were determined in the cell culture supernatants by plaque test. Data are shown as average ± SD of inhibition of viral replication (% of control) from two independent biological replicates, each performed in technical triplicates. The IC₉₀ values were determined by non-linear regression.

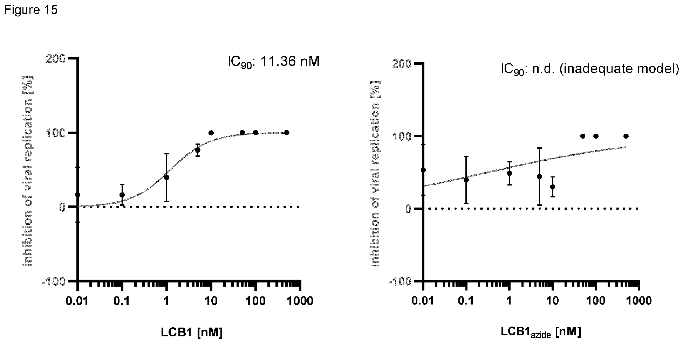

Figure 15 shows the determination of the IC₉₀ of the minibinders LCB1 and LCB1_{azide} in a SARS-CoV-2 neutralization assay. Calu-3 cells were infected with SARS-CoV-2 HH01 at an MOI of 0.01 and therapeutically treated with LCB1 and LCB1_{azide} at eight different concentrations (range 0.01 to 500 nM). Non-treated cells were used as negative control. At 48h post infection, infectious virus titers were determined in the cell culture supernatants by plaque test. Data are shown as average ± SD of inhibition of viral replication (% of control) from one biological replicate, performed in technical triplicates. The IC₉₀ values were determined by non-linear regression.

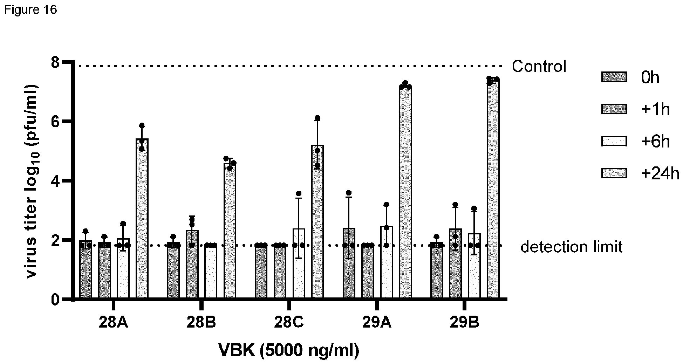

Figure 16 shows the inhibition of SARS-CoV-2 HH01 replication by VBKs 28A-C and 29A-B when added at various time points after infection. Calu-3 cells were infected with the SARS-CoV-2 HH01 at an MOI of 0.01 and therapeutically treated with the VBKs at a single-dose of 5 µg/ml at 0h (*i.e*., directly after virus infection), 1h, 6h an 24h post infection. Non-treated cells were used as negative control (Control). At 48h post infection, infectious virus titers were determined in the cell culture supernatants by plaque test. Viral titers are shown as average log10 plaque-forming units/ml ± SD from one biological replicate, performed in technical triplicates. The detection limit is indicated with a dashed line.

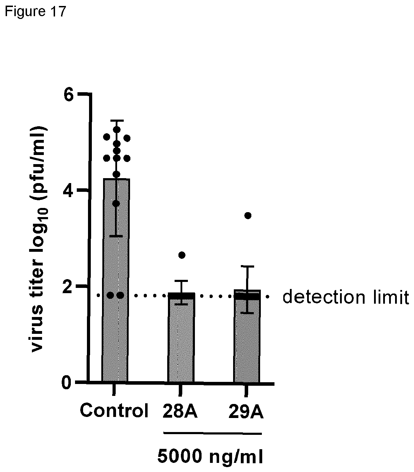

Figure 17 shows the inhibition of SARS-CoV-2 HH01 replication by VBKs 28A and 29A on primary human lung cells. Human bronchial epithelial primary cells (HBEpC) were differentiated in the air-liquid interface (ALI) system and infected with SARS-CoV-2 HH01 at an MOI of 1 from the apical site. Subsequently, cells were therapeutically treated with the VBKs 28A and 29A at a single-dose of 5 µg/ml in the basal medium. Non-treated cells were used as negative control (Control). At 48h post infection, infectious virus titers were determined in the apical cell culture supernatants by plaque test. Viral titers are shown as average log10 plaque-forming units/ml ± SD from one biological replicate, performed with *n* = 12 technical replicates per condition. The detection limit is indicated with a dashed line.

Figure 18 shows the schematic representation of the syntheses of the PLGA modifications used in the nanoparticles.

Figure 19 shows the modification of the nanoparticle surfaces via thiol-maleimide- and azide-alkyne Click reactions.

SEQ ID NO: 1 shows the amino acid sequence of N-terminal extension MSYYHHHHHHDYDIPTTENLYFQGSGSGGS of recombinant core LCB 1 and AHB2 minibinders.

SEQ ID NO: 2 shows the amino acid sequence of the C-terminal extension GSGGSGSGC of recombinant core LCB 1 and AHB2 minibinders.

### Examples

### Methods

### Production and purification of recombinant SARS-CoV-2 spike RBD minibinders

Codon optimized synthetic genes encoding LCB1 and AHB2 were cloned into the vector pET28H6TEV (modified pET28b vector encoding an N-terminal 6xHis-tag followed by a tobacco etch virus (TEV) protease site). Recombinant core LCB1 and AHB2 minibinders harboring N-terminal (MSYYHHHHHHDYDIPTTENLYFQGSGSGGS) (SEQ ID NO: 1) and C-terminal (GSGGSGSGC) (SEQ ID NO: 2) extensions were produced in *E. coli* BL21(DE3) cells (New England Biolabs) by auto induction (Overnight Express Instant TB Medium, Novagen) at 25 °C. Bacterial cells were then harvested by centrifugation (10 500 × *g*, 20 minutes at 16 °C) and stored at -80 °C. Frozen bacterial cells were resuspended in lysis buffer (100 mM HEPES, 300 mM NaCl, 1 mM AEBSF, pH 7.5) and disrupted at 1000 bar using a high-pressure homogenizer (Emulsiflex C5, Avestin). After centrifugation (48,000 × *g*, 20 minutes at 4 °C), His-tagged proteins were purified by metal chelate affinity chromatography using a 20 ml TALON Superflow column (Clontech). Proteins were eluted from the TALON column with 20 mM HEPES, 300 mM NaCl, 200 mM imidazole, pH 7.5, incubated overnight with TEV protease (made in-house, 4 µg TEV protease/1 mg minibinder) at room temperature and further purified by size exclusion chromatography (SEC) on a Superdex 75 prep grade 26/600 column (Cytiva) in 50 mM HEPES, 150 mM NaCl, pH 7.5. SEC fractions containing pure LCB1 and AHB2 were snap frozen in liquid N₂ and stored at -80 °C.

### Synthesis of LCB1-azide

To produce LCB1-azide, 546 µl of TCEP stock solution (273 µmol, 0.5 M adjusted to pH 7 with NaOH) were added to 10 ml ofLCB 1 solution (2.17 mg/ml, 273 µM, 2.73 µmol in 50 mM HEPES buffer containing 150 mM NaCl, pH 7.5). After brief vortexing and a reaction time of 30 min, a buffer exchange using oxygen-free sodium borate buffer (50 mM, pH 9) was performed under anaerobic conditions by means of size exclusion chromatography (SEC) employing four Cytiva sephadex PD-10 size exclusion columns. Four fractions of 3 ml of LCB1 solution in borate buffer were obtained and combined. 27 mg (79.6 µmol) of Bromoacetamido-PEG3-N₃ (Sigma Aldrich, Prod.no. QBD11217, Merck KGaA, Darmstadt, Germany; M = 339.19 g/mol; protein : linker = 1 : 29.2) was added to the LCB1 solution. The reaction mixture was vortexed at 500 rpm for 2.5 h at 25 °C. Afterwards, the solution was split into five fractions of 2.5 ml and each fraction was added to one SEC column preconditioned with deionized sterile water. The protein was eluted from the columns by adding 3 ml of MiliQ water to each column. The protein fractions from all three columns were combined. 15 ml of LCB 1-azide solution (138.9 µM, 2.086 µmol, 17.1 mg) in MilliQ water were obtained. The protein was snap frozen and lyophilized.

### Surface plasmon resonance measurements

Real-time analyses were performed on a Biacore T200 system at 25 °C using CM5 sensor chips (Cytiva). Prior to ligand immobilization, sample and reference flow cells surfaces were activated by injecting a 1:1 mixture of 0.4 M EDC and 0.1 M NHS at a flow rate of 10 µl/min. Human ACE2 (Sino Biological 10108-H08H) was diluted in 10 mM sodium acetate, pH 4.5 (5 µg/ml), and was covalently coupled to a level of about 500 response units (RU). Excess reactive succinimide esters were deactivated with 1 M ethanolamine-HCl, pH 8.5. After amine coupling of 0.1 M ethylenediamine in 0.1 M sodium borate, pH 8.5, reduced (100-fold molar excess of TCEP) and diluted minibinders LCB1 and AHB2 (5 µg/ml in 10 mM sodium acetate, pH 4.0) were immobilized to a level of around 50 RU by standard maleimide coupling using 50 mM sulfo-GMBS (N-γ-maleimidobutyryl-oxysulfosuccinimide ester, Thermo Scientific) in 0.1 M sodium borate, pH 8.5. Excess maleimide groups were blocked with 50 mM cysteine, 1 M NaCl in 0.1 M sodium acetate, pH 4.0.

Recombinant Spike RBDs from SARS-CoV-2 and VOCs were purchased from Sino Biological (WH-1 RBD, 40592-V08B; WH-1 RBD-mFc, 40592-V05H; BA.1 RBD, 40592-V08H121; BA.1 RBD-mFc, 40592-V05H3; BA.5 RBD, 40592-V08H131), reconstituted in water and transferred in HBS-EP+ assay buffer (10 mM HEPES, 150 mM NaCl, 3 mM EDTA, 0.05% (v/v) Surfactant P20, pH 7.4) using PD MiniTrap G-25 desalting columns (Cytiva). Serial twofold dilutions of each RBD were injected in single cycle mode at a flow rate of 30 µl/min. Flow cells loaded with minibinders were regenerated after each single cycle with a 30-second pulse of 6 M guanidinium chloride. Reference-subtracted raw data were processed und fitted to a 1:1 interaction model using Biacore Evaluation Software 3.2.0.5.

The following modifications were necessary to immobilize LCB1-azide on the sensor chip. Prior to immobilization of LCB1-azide, EDC/NHS activated flow cells were treated with the amine functionalized cyclooctyne derivative (*N*-[(1R,8S,9s)-Bicyclo[6.1.0]non-4-yn-9-ylmethyloxycarbonyl]-1,8-diamino-3,6-dioxaoctane (BCN-amine, Sigma). BCN-amine (0.3 mM in 10 mM maleic acid, pH 6.0) was injected and covalently coupled to a level of about 400 RU, followed by deactivation of remaining reactive succinimide esters with 1 M ethanolamine-HCl, pH 8.5. LCB1-azide (10 µg/ml in 10 mM sodium acetate, pH 4.0) was bound by click coupling to a level of around 60 RU.

### Formulation of particle suspensions Set_1

A single emulsion technique was used for the NP formulation utilizing a T 10 basic ULTRA-TURRAX^{®} (IKA, Staufen, Germany). Each mixture of polymers was dissolved in ethyl acetate with a concentration of 10 mg/ml. 2 ml of the organic solution were mixed at a stage of around 4.5 with 1 ml of an aqueous phase containing 1% (w/v) of poly(vinyl alcohol) (PVA) for five minutes. To stabilize the particle suspension, 14 ml of 10 mM HEPES buffer pH 6 were added. The suspension was left at room temperature under continuous stirring at 800 rpm to evaporate the organic solvent for at least four hours.

The particles were purified by ultrafiltration using a 5804 R centrifuge (Eppendorf, Hamburg, Germany). They were transferred to an Amicon^{®} Ultra 15 ml Centrifugal Filter (100,000 g mol⁻¹ MWCO) and centrifuged in a swing bucket rotor with 4000 × g at 4 °C for 20 minutes. Then the remaining volume of the sample was added and again the suspension was centrifuged for 30 min until the retentate volume in the filter was at or below 1500 µl. The nanoparticles (NPs) were resuspended and 5 ml of 10 mM HEPES buffer pH 6 was added to the retentate NP suspension. This washing step was repeated four times with 15 minutes of centrifugation and 5 ml HEPES buffer added after each centrifugation. For the last purification step the NPs were centrifuged for 19 minutes until a retentate volume of 500 µl. The NP suspension was collected. In order to lose as little NPs as possible, the filter membrane of the Amicon^{®} was rinsed with HEPES buffer to collect the NP adhering to the filter membrane and was transferred to the NP suspension. The volume of the NP suspension was adjusted to a final of 2 ml and a polymer concentration of 10 mg/ml. After each purification step the total volume of washing water was collected.

In order to remove DTT from the minibinder LCB1, a buffer exchange was performed using PD MidiTrap G-25 (Cytiva) and PD-10 desalting columns (Cytiva) in accordance with the manufacturer's manual against 50 mM NaH₂PO₄, 150 mM NaCl pH 7.5. Using a NanoDrop plate the absorbance of the minibinder LCB1 was measured at 280 nm and the concentration of the peptide was calculated using the molar extinction coefficient (ε) and Lambert-Beer law. A 100-fold molar excess of TCEP pH 7.0 was added to the minibinder and incubated for 20 minutes at room temperature to reduce potential disulfide bonds.

For the maleimide coupling the calculated amount of minibinder LCB1 for a ratio of 2:1 (n_{maleimide moiety polymer}:n_{minibinder}) was added to 1 ml of the purified NP suspension and incubated at 200 rpm and room temperature for 20 h. The amount of minibinder for the control sample S was set to be the same amount as particle M.

Similar to the first purification step, the NP suspension was purified via ultrafiltration, transferred to an Amicon^{®} Ultra 15 ml Centrifugal Filter (100,000 g/mol MWCO) and centrifuged in a swing bucket rotor with 4000 × *g* at 4 °C for 7 minutes. After resuspension of the NPs, 5 ml of 10 mM HEPES buffer pH 6 was added. This washing step was repeated two times with 15 minutes of centrifugation and 5 ml HEPES buffer added after each centrifugation. For the last purification step, the NPs were centrifuged for 21 minutes until a retentate volume of 500 µL. The NP suspension was collected. In order to lose as little NPs as possible, the filter membrane of the Amicon^{®} was rinsed with HEPES buffer to collect the NP adhering to the filter membrane and was transferred to the NP suspension. The volume of the NP suspension was adjusted to a final of 5 ml and a polymer concentration of 2 mg/ml. After each purification step, the total volume of washing water was collected.

### Formulation of particle suspensions Set_2 - VBKs 28 A-C

The formulation of the NPs was carried out by a single emulsion technique using a T 10 basic ULTRA-TURRAX^{®} (IKA, Staufen, Germany). Each mixture of polymers was dissolved in ethyl acetate to a final concentration of 10 mg/ml. One ml of an aqueous phase containing 1% (w/v) of poly(vinyl alcohol) (PVA) was mixed at a stage of around 4.5 with 2 ml of the organic solution for 5 minutes. To stabilize the particle suspension 14 ml of 10 mM HEPES buffer pH 6 were added. To evaporate the organic solvent the suspension was left at room temperature under continuous stirring at 800 rpm for at least four hours.

The particles were purified by ultrafiltration using a 5804 R centrifuge (Eppendorf, Hamburg, Germany). They were transferred to an Amicon^{®} Ultra 15 ml Centrifugal Filter (100,000 g/mol MWCO) and centrifuged in a swing bucket rotor with 2000 × *g* at 4 °C for 20 minutes. Then the remaining volume of the sample was added and again centrifuged for 33 minutes until the retentate volume in the filter was at or below 1500 µl. The NPs were resuspended and 5 ml of 10 mM HEPES buffer pH 6 was added to the retentate NP suspension. This washing step was repeated for a total of six times. The NPs were centrifuged for 23, 15 and two times 20 minutes and resuspended with always 5 ml HEPES buffer after each centrifugation until they were finally for the last purification step centrifuged for 30 minutes until a retentate volume of 500 µL. The NP suspension was collected. In order to lose as little NPs as possible, the filter membrane of the Amicon^{®} was rinsed with HEPES buffer to collect the NP adhering to the filter membrane and was transferred to the NP suspension. The volume of the NP suspension was adjusted to a final of 2 ml and a polymer concentration of 10 mg/ml. After each purification step the total volume of washing water was collected.

For the maleimide coupling the calculated amount of minibinder LCB1 for a ratio of 4:1 (n_{maleimide moiety polymer}:n_{minibinder}) was added to 1 ml of the purified NP suspension and incubated at 200 rpm and room temperature for 20 hours. The amount of minibinder for the control samples 28B and 28C was set to be the same amount as particle 28A.

Similar to the first purification step the NP suspension was purified *via* ultrafiltration, transferred to an Amicon^{®} Ultra 15 ml Centrifugal Filter (100,000 g/mol MWCO) and centrifuged in a swing bucket rotor with 2000 × g at 4 °C for 8 minutes. After resuspension of the NPs 5 ml of Milli-Q water was added. This washing step was repeated for a total of four times. The NPs were centrifuged for 16 and 12 minutes and were resuspended with always 5 ml Milli-Q water until they were finally for the last purification step centrifuged for 12 minutes until a retentate volume of 500 µl. The NP suspension was collected. In order to lose as little NPs as possible, the filter membrane of the Amicon^{®} was rinsed with Milli-Q water to collect the NP adhering to the filter membrane and was transferred to the NP suspension. The volume of the NP suspension was adjusted to a final of 5 ml and a polymer concentration of 2 mg/ml. After each purification step the total volume of washing water was collected.

### Formulation of particle suspensions Set_2 - VBKs 29A-B

The formulation of the NPs was carried out by a single emulsion technique using a T 10 basic ULTRA-TURRAX^{®} (IKA, Staufen, Germany). Each mixture of polymers was dissolved in ethyl acetate to a final concentration of 10 mg/ml. 1 ml of an aqueous phase containing 1% (w/v) of poly(vinyl alcohol) (PVA) was mixed at a stage of around 4.5 with 2 ml of the organic solution for 5 minutes. To stabilize the particle suspension 14 ml of Milli-Q water were added. To evaporate the organic solvent the suspension was left at room temperature under continuous stirring at 800 rpm for at least four hours.

The particles were purified by ultrafiltration using a 5804 R centrifuge (Eppendorf, Hamburg, Germany). They were transferred to an Amicon^{®} Ultra 15 ml Centrifugal Filter (100,000 g/mol MWCO) and centrifuged in a swing bucket rotor with 2000 × *g* at 4 °C for 20 minutes. Then the remaining volume of the sample was added and again with for 13 minutes until the retentate volume in the filter was at or below 1500 µL. The NPs were resuspended and 5 ml of Milli-Q water was added to the retentate NP suspension. This washing step was repeated for a total of six times. The NPs were centrifuged three times for 8 minutes and then for 7 minutes and resuspended with always 5 ml Milli-Q water after each centrifugation step until they were finally for the last purification step centrifuged for 7 minutes until a retentate volume of 500 µL. The NP suspension was collected. In order to lose as little NPs as possible, the filter membrane of the Amicon^{®} was rinsed with Milli-Q water to collect the NP adhering to the filter membrane and was transferred to the NP suspension. The volume of the NP suspension was adjusted to a final of 2 ml and a polymer concentration of 10 mg/ml. After each purification step the total volume of washing water was collected.

For the alkyne coupling the calculated amount of minibinder LCB1-azide for a ratio of 4:1 (n_{maleimide moiety polymer}:n_{minibinder}) was added to 1 ml of the purified NP suspension and incubated at 200 rpm and room temperature for 20 h. The amount of minibinder for the control samples 29B was set to be the same amount as particle 29A.

Similar to the first purification step the NP suspension was purified *via* ultrafiltration, transferred to an Amicon^{®} Ultra 15 ml Centrifugal Filter (100,000 g/mol MWCO) and centrifuged in a swing bucket rotor with 2000 × *g* at 4 °C for 8 minutes. After resuspension of the NPs 5 ml of Milli-Q water was added. This washing step was repeated for a total of four times. The NPs were centrifuged for 16 and 12 minutes and were resuspended with always 5 ml Milli-Q water until they were finally for the last purification step centrifuged for 12 minutes until a retentate volume of 500 µL. The NP suspension was collected. In order to lose as little NPs as possible, the filter membrane of the Amicon^{®} was rinsed with Milli-Q water to collect the NP adhering to the filter membrane and was transferred to the NP suspension. The volume of the NP suspension was adjusted to a final of 5 ml and a polymer concentration of 2 mg/ml. After each purification step the total volume of washing water was collected.

### Scanning Electron Microscopy (SEM)

Another method for the investigation of NPs is scanning electron microscopy (SEM). For the imaging of the particles, a Sigma VP Field Emission Scanning Electron Microscope (Carl-Zeiss, Jena, Germany) with an Inlens detector and an accelerating voltage of 5 to 6 kV was used. Therefore, the samples were previously coated with a 4 nm platinum layer by using a CCU-010 HV sputter (Safematic, Zizers, Switzerland). For the evaluation of the NPs sizes, ImageJ was used.

### Bicinchoninic acid assay (BCA assay)

The LCB1 and LCB 1-azide concentration of the NP formulation was quantified using a Micro BCA^{™} Protein Assay Kit (Thermo Fisher Scientific Inc.). The assay was performed following the manufacturer's protocol and their microplate procedure in a VWR^{®} Tissue Culture 96 wells-F plate. The absorption was measured using the Infinite M200 Pro plate reader (Tecan Group, Männedorf, Switzerland) at a wavelength of λ = 562 nm 3 × 3 multiple reads per well and a well border of 2000 µm. A calibration curve for the LCB1 in a range of 963.71 to 0.47 µg/ml and the LCB 1-azide in a range of 1250.02 to 0.31 µg/ml was created. Depending on the aqueous phase either 10 mM HEPES buffer pH 6 or Milli-Q water was used as blank value for the calibration curve, the NP suspensions and the washing water.

### Dynamic light scattering (DLS)

The characterization of the particles in terms of size and polydispersity was performed by DLS measurements using a Zetasizer Nano ZS with a laser wavelength of λ = 633 nm (Malvern Panalytical GmbH, Malvern, UK). Each measurement of the size and polydispersity index (PDI) was implemented in UV cuvettes consisting of polystyrene (Brand GmbH + Co KG, Wertheim, Germany) with five runs of 30 seconds after an equilibration time of 30 seconds, while the backscatter angle was set at 173°. The size distribution by intensity was used to gather the hydrodynamic diameter (d_{H}) of the NPs. DLS measurements were performed after the formulation of the NPs, after each purification step, after different times of storage at 4-8 °C.

### PVA assay

For the quantification of the PVA residue in the NP formulation, a PVA assay was performed *via* UV-Vis spectroscopy. Due to the complex formation of PVA with iodine of a Lugol solution, the absorption is measurable at λ = 650 nm. One freeze-dried aliquot was used and resuspended in 1 ml pure water. The 90 µL of the resuspended suspension was transferred into a VWR^{®} Tissue Culture 96 wells-F plate. Afterward, 20 µL of 1 M sodium hydroxide was added to each well, and the mixture was incubated for 15 min by using a BioShaker with 850 rpm at room temperature to degrade the particles via hydrolyzation of the NP matrix. Subsequently, 20 µL of a 1 M hydrochloric acid was added for neutralization. The complexation started after adding 60 µL 0.65 M boric acid and 10 µL Lugol solution. Next, the sample absorption was measured within 15 min with the plate reader. The associated calibration curve was set up analogously to the NP samples. The assay was performed with the different NP suspensions.

### Cells

Human lung adenocarcinoma epithelial cells (Calu-3; *ATCC-HTB-55*) were cultured in Eagles Minimum Essential Medium (EMEM), supplemented with 10 % (v/v) fetal bovine serum (FBS) and 1 % penicillin/streptomycin (P/S). African green monkey kidney epithelial cells (VeroE6; *ATCC-CCL-81*) were cultivated in Dulbecco's Modified Eagles Medium (DMEM) medium, supplemented with 10 % (v/v) FBS, 1 % (w/v) L-glutamine (L-Glu) and 1 % (w/v) P/S. Human bronchial epithelial primary cells (HBEpC) were purchased from Promocell and cultivated in complete airway epithelial cell growth medium (Promocell). The murine macrophage cell line RAW264.7 (ATCC TIB-71) was cultivated in DMEM (Gibco) supplemented with 10 % (v/v) fetal bovine serum (FBS) (GE Healthcare Life Sciences), 1 % (w/v) ultraglutamine (Gibco) and 27.5 µg/ml gentamicin sulfate (Gibco). at 37 °C, 5 % (v/v) CO₂ in a humidified chamber.

All cell lines were maintained in a temperature-controlled incubator (37 °C, 5 % (v/v) CO₂, in a humidified atmosphere) and regularly verified to be mycoplasma negative.

### Viruses

The wild type/Wuhan-like SARS-CoV-2 (SARS-CoV-2/Germany/HPI06-n/2020, HH01) was isolated from a nasal swab of a SARS-CoV-2 infected patient who was treated in an ICU at the University Medical Campus Hamburg-Eppendorf, Hamburg, Germany. Delta and Omicron BA.1 SARS-CoV-2 isolates (SARS-CoV-2/B.1.617.2/Germany/Hamburg/01/2021 and SARS-CoV-2/B.1.1.529/Germany/Hamburg/ 01/2021, respectively) were obtained from the University Medical Campus Hamburg-Eppendorf, Hamburg, Germany. SARS-CoV (Frankfurt 1, FFM) was a kind gift from Dr. B. L. Haagmans (Erasmus Medical Center, Rotterdam, The Netherlands), and MERS-CoV (EMC-2012) was a kind gift from Prof. Dr. Christian Drosten (Charité Universitätsmedizin Berlin, Berlin, Germany). All coronavirus stocks used in this study were generated by propagation on VeroE6 cells, and viral titers were determined by plaque test following established protocols.

### Therapeutics

The VBKs tested for their ability to inhibit SARS-CoV-2 replication in Calu-3 and HBEpC cells were stored at 4 °C (particles M through T) or -20 °C (particles 28A-C and 29A-B). Remdesivir was obtained from Hölzel Diagnostika Handels GmbH (Cologne, Germany). Remdesivir stock solutions were prepared in DMSO and stored at -80 °C. For all therapeutics, repeated freeze-thaw circles were avoided whenever possible.

### Air-liquid interface (ALI) differentiation of human bronchial epithelial primary cells (HBEpC)

HBEpC cells were seeded on porous 24-well transwell inserts (0.4 µM, polyester (PET) membrane) with 0.25 × 10⁵ cells per well. Cells were exposed to medium from both the apical and basal site and cultivated for 5 to 7 days at 37 °C (5% (v/v) CO₂, 96% rH) until they reached 100% confluence (as estimated by microscopic analysis). At this time, the medium from the apical site was removed and the cells were exposed to air from the apical site and medium (complete air liquid interface medium; Promocell) from the basal site in order to initiate the differentiation process. The medium in the basal chamber was changed every 2-3 days. After approximately two weeks of differentiation, the cells were also washed twice per week with phosphate-buffered saline (PBS) to remove mucus. Simultaneously, transepithelial electrical resistance (TEER) measurements were carried out once per week to assess the integrity of the cell monolayer. At four to five weeks post air exposure, the differentiation process was completed (as indicated by high TEER values and pronounced mucus production), and the ALI cultures were subjected to SARS-CoV-2 infection.

### Cell viability assay

Calu-3 cells were seeded in 96-well plates with 3.5 × 10⁵ cells/well, for 24 h. The compounds to be tested or the VBK solvent as positive control were serially diluted in growth medium to obtain 5-fold of the desired final concentrations. The growth medium was removed from the cells and replaced with 80 µl per well of fresh growth medium. Subsequently, 20 µl of the diluted compounds were added in quadruplicates for each concentration (i.e. 5-fold dilution to reach the final concentrations). Cells were incubated for 48 h at 37 °C (5% (v/v) CO₂, 96 % rH). At 48 h post treatment, cell viability was measured on a *Tecan Safire 2* plate reader using the *CellTiter 96*^{®} *Non-Radioactive Cell Proliferation Assay (MTT)* (Promega) according to manufacturer's instructions.

### Cell cytotoxicity assay

Calu-3 cells were seeded in 96-well plates and treated with the VBKs to be tested (or their solvent as control) as described above (*"Cell viability assay"*)*.* At 48h post treatment, cell cytotoxicity was measured on a *Tecan Safire 2* plate reader using the *CytoTox 96*^{®} *Non-Radioactive Cytotoxicity Assay* (Promega) according to manufacturer's instructions. This assay is based on the release of intracellular lactate dehydrogenase (LDH) into the cell culture supernatant when the cell membrane becomes damaged.

### Phagocytosis Assay

RAW264.7 cells were seeded with a density of 2×10⁵ cells/well in a 24-well plate with cover slips one day in advance. At the following day, prior to infection RAW264.7 cells were prestained with CTB-Alexa 555 (Invitrogen) to label the sphingolipid GM1 as part of the cytoplasmic membrane as well as intracellular membranes. Afterwards, macrophages were infected with 10 µg/ml VBKs and VSVΔG-S_{Atto643} regarding an MOI of 0.1 or with VSVΔG-S_{Atto643} only. After a synchronization of infection, the co-incubation was continued for two or four hours, and the samples were fixed for ten minutes with 3.7% (v/v) formaldehyde/PBS. The samples were microscoped with an LSM 780 (Zeiss) and pictures were analyzed by Zen software.

### Flow cytometry

To check the ability to bind the SARS-CoV-2 RBD the formulated VBKs were tested by labeling with a recombinant SARS-CoV-2 RBD-Alexa Fluor 647 (R&D Systems) by flow cytometry. Samples with unstained nanoparticles as well as nanoparticles without minibinder were analyzed. For sample preparation, 100 µg/ml VBKs were incubated with 1 µg SARS-CoV-2 RBD-Alexa Fluor 647/PBS for 30 minutes. After washing by centrifugation (250 × *g* for five minutes at RT) the particles were resuspended in PBS (Gibco, Thermo Fisher Scientific Inc.) and measured with a BD LSRFortessa. Evaluation of the obtained data was performed using FlowJo.

### Viral replication kinetics

All experiments with live SARS-CoV-2 isolates were performed under BSL-3 conditions at the Leibniz Institute of Virology (Hamburg, Germany) following standard operating procedures. Calu-3 cells were seeded in 24-well plates with 3.5 × 10⁵ cells/well, for 24 h. The VBKs to be tested were diluted in infection medium (DMEM, supplemented with 2% (v/v) FBS, 1% (w/v) L-Glu and 1% (w/v) P/S) to reach the final concentrations. The growth medium was removed from the cells, cells were washed once with PBS and subsequently inoculated with SARS-CoV-2 at a MOI (multiplicity of infection) of 0.01, prepared in infection medium. After attachment of viral particles to the cells for 45 min, the inoculum was removed, cells were washed twice with 1x PBS, and infection medium containing the test compounds was added (1 ml/well). For the delayed treatment experiment, VBKs at the desired concentration were either directly added to the cells ("0h" time point), or added at 1 h, 6 h or 24 h post infection directly into the medium. As SARS-CoV-2 replication peaks at approximately 48 h post infection (p.i.), this time point was chosen for all subsequent analyses. At 48 h p.i., supernatants were collected from infected cells and stored at -80 °C.

ALI-differentiated HBEpC cells (24-well format) were infected with SARS-CoV-2 from the apical site at a MOI of 1 according to the procedures described above. ALI medium containing the test compounds was added to the basal chamber. At 48h p.i., 100 µl of ALI medium was added to the apical site, cells were incubated at 37 °C for 15 min to harvest virus particles present in the mucus, and the supernatant was stored at -80 °C. Viral titers in all cell culture supernatants were determined by plaque test on VeroE6 cells as described below.

### Plaque test

Viral titers in supernatants collected from SARS-CoV-2 infected cells were determined by plaque test on VeroE6 cells. Briefly, VeroE6 cells were seeded in 12-well plates (2.5 × 10⁵ cells per well) for 24 h. Cell culture supernatants were 10-fold serially diluted in PBS. The growth medium was removed from the cells, cells were washed once with PBS, and diluted supernatants were added (150 µl/well). After 30 min inoculation, an overlay medium (double-concentrated minimal essential medium (MEM; supplemented with 2 % (w/v) L-glu, 2 % (w/v) P/S, 0.4 (w/v) % bovine serum albumin (BSA)), mixed 1: 1 with 2.5 % avicel solution (prepared in ddH₂O)) was added to the cells (1.5 ml/well). Then, cells were incubated for 72 h at 37 °C. After 72 h, the overlay medium was removed from the cells, and following a washing step with PBS the cells were fixed with 4 % (v/v) paraformaldehyde (PFA) for at least 30 min at 4 °C. Subsequently, the PFA solution was removed, and the cells were counterstained with crystal violet solution to visualize the virus-induced plaques in the cell layer. The number of plaques at a given dilution was used to calculate the viral titers as log10 plaque-forming units per ml (PFU/ml).

### Spike inhibitor colorimetric competition assay

The assay was performed according to the protocol of product #78365 "Spike Trimer (S1+S2) (B.1.1.529 BA.1, Omicron Variant) (SARS-CoV-2): ACE2 Inhibitor Screening Colorimetric" by PBS bioscience (San Diego, CA, USA). In order to obtain the results for the wt-spike protein, the assay was performed using the wt-spike variant (product #100728; BPS bioscience, San Diego, CA, USA) instead of the omicron BA.1 variant that is originally shipped with this assay (product #101343; BPS bioscience, San Diego, CA, USA).

A spike trimer solution (4 µg/ml in PBS, 50 µl/well; either the wt-variant or the omicron BA.1 variant) was used to coat a clear, polypropylene (PP) flat-bottom 96-well plate with the spike trimer by incubation for 20 h at 5 °C. After removal of the spike solution and washing three times with 100 µl/well of immuno-buffer (provided by the manufacturer), each well was blocked by incubation with blocking buffer (provided by the manufacturer) for 1 h. The blocking buffer was removed, and the plate was tapped upside down onto clean paper towels to remove excess liquid. A fourfold dilution series of LCB1-azide in blocking buffer (4 µM - 1 pM) was created and 50 µl of diluted minibinder were given to each well labeled "test inhibitor". 50 µl of blocking buffer were added to the wells labeled "blank" and "positive control". The plate was incubated for 1 h at 22 °C with mild agitation. 50 µl of biotin-labeled ACE2 (ACE2-biotin; 1.5 µg/ml in blocking buffer) was given to each well labeled "test inhibitor" or "positive control". The final concentration of LCB1-azide was between 2 µM and 0.5 pM, the final concentration of ACE2-biotin was 0.75 µg/ml which corresponds to approximately 7.5 nM. 50 µl of blocking buffer were added to each well labeled "blank". Following 1 h of incubation at 22 °C with mild agitation, the solution was discarded, and the wells were washed three times with 100 µl of immuno-buffer. Excess buffer was removed by tapping the plate upside down on a clean paper towel. The streptavidin-HRP solution provided by the manufacturer was diluted 1000-fold using blocking buffer. Of this diluted streptavidin-HRP solution, 50 µl were added to each well. After an incubation period of 45 min, the plate was washed three times with 100 µl of immuno-buffer. Substrate (50 µl) was added to all wells. Substrate turnover was quick and complete after approximately two minutes as determined by measuring every other PC and Blank well using a Tecan spark plate reader (Tecan Group AG, Männedorf, Switzerland) at 450 nm.

In case of the omicron BA. 1-related experiment, the reaction was stopped after two minutes by addition of 100 µl of 1 M HCl to each well, which brought an instantaneous color change from light blue to intense yellow. The absorbance of each well was measured at 450 nm using the Tecan spark plate reader.

By accident, in case of the wt-spike-related experiment the reaction was not stopped until after 12 min. Addition of 1 M HCl (100 µl) to each well brought an instantaneous color change from light blue to intense yellow. The absorbance of each well at 450 nm was too strong to be accurately determined by the Tecan spark plate reader. Therefore, aliquots were taken out of each well and pipetted into a corresponding well of a new flat-bottom 96-well plate. After 16.7-fold dilution with water, the absorption of each well of this diluted plate was measured with the Tecan Spark plate reader at 450 nm.

The data was processed by blank subtraction and normalization so that 1 would indicate no inhibition, while 0 represents complete inhibition. A generalized logistic function was fitted to the data points to determine the IC₅₀ values.

### Statistical analysis

All data were analyzed using Microsoft Excel and GraphPad Prism software. Non-linear regression was performed to determine the inhibitory concentrations 50 and 90 (IC₅₀ and IC₉₀, respectively) of the 8-dose treatments on Calu-3 cells.

### Production and biophysical characterization of minibinders targeting SARS-CoV-2

The use of structural entities that bind the SARS-CoV-2 spike protein with nanomolar to picomolar affinity is crucial for the success of our strategy as the nanoparticles are required to outcompete the hACE2 receptor for binding of virions. The minibinder on the surface of nanoparticles needs to be able to interact with the wild-type (wt) spike RBD with nanomolar affinity. Initially, the inventors considered the binding entities to be short peptides resembling the hACE2 amino acid sequence, but thorough investigations indicated that the affinity of peptides for the spike RBD was generally too low for the intended use.

Therefore, the inventors turned to so-called "hyperstable minibinders" [8, 28] consisting of small proteins designed to inhibit the interaction of the spike RBD with hACE2 by blocking the RBD binding site with picomolar affinity and high specificity. Two of these minibinders, namely LCB 1 and AHB2 [8] were produced in *E. coli* with an N-terminal His₆-tag. After purification using Co-chelate chromatography, removal of the His₆-tag by a tobacco etch virus (TEV) protease and subsequent size exclusion chromatography, the minibinders LCB1 and AHB2 were obtained in yields of 109 mg and 40 mg per liter culture, respectively (Figure 1). Both minibinders were flanked by short sequences of Ser and Gly at their termini and possess a Cys residue at their C-terminus for covalent attachment to the nanoparticle.

Surface plasmon resonance (SPR) spectroscopy was employed to assess the minibinder's affinity for the spike RBD of the wild-type SARS-CoV-2 as well as that of the omicron (PANGO lineage B.1.1.529) variants BA.1 and BA.5. As the minibinders need to be immobilized on the surface of nanoparticles, we decided to use the minibinders as ligands, *i.e.* immobilized at the surface of the SPR chip. To attach the minibinders, the chip was modified with cysteine-reactive groups. In addition to using monomeric RBDs as an analyte, we also used dimeric RBDs fused together *via* a crystallizable fragment (Fc) region of a murine (m) immunoglobulin G1 (IgG1) antibody. The monomeric RBD has the advantage to interact with a minibinder in a monovalent fashion which is easy to interpret, but it suffers from rather poor solubility and the tendency to aggregate. In contrast, the mFc-tagged dimeric RBD (RBD-mFc) is highly soluble but in principle allows divalent interactions when the chip surface is densely covered with minibinders. As divalent interactions would be more difficult to fit and to interpret, the SPR chip was loaded with a relatively low densities of minibinder to reduce divalent interactions as much as possible. In general, the dimeric RBDs were found to have similar or slightly higher affinities for the immobilized minibinders than their monomeric counterparts. When comparing the affinity of minibinders LCB1 and AHB2 for the monomeric wt-RBD, LCB1 displayed an approximately 10-fold higher affinity for the wt-RBD (K_{D} ≈ 5 pM) than AHB2 (K_{D} ≈ 50 pM). The affinity of LCB 1 and AHB2 for the omicron BA.1 and BA.5 RBDs are comparable and lie in the low nanomolar range. Because of its extremely high affinity, the LCB1 minibinder was chosen for the experimental functionalization of nanoparticles.

### Synthesis of functional PLGA

The nanoparticles formulated in this invention were made of poly(lactic-co-glycolic acid) (PLGA), which is often used due to its well documented biocompatibility and biodegradability. The monomers lactide acid and glycolic acid can be metabolized by the body, and by varying the monomer ratios it is possible to influence the degradation rate. Thus, with increasing PLA content, degradation can be decelerated. In addition, PLGA is FDA approved and is already used in authorized drugs [9]. In this invention, PLGA with molar mass (M_{w}) of 7000 to 17000 g/mol and a PLA to PGA ratio of 50:50 was used. Due to the possibility of opsonization and recognition by the immune system, besides PLGA a blockcopolymer containing PLGA and poly(ethylene glycol) (PEG) as a stealth polymer was also used for the nanoparticles (see Figure 18). PEGs with molar masses between 1-5 kDA are usually used to particularly exert the stealth effect on nanoparticle surfaces, which led to the choice of PEG with 2.1 kDa in this invention [10]. Upon formulation, the hydrophilic PEG should be located towards the outside of the particle, thus, the *ω*-end groups should be found on the surface of the particle facilitating the modification with functional peptides. The modification of the *ω*-end group of the PLGA/PEG-PLGA was done by an ester activation using *N*-hydroxysuccinimide (NHS) with subsequent amidation using maleimide- or PEG-maleimide amine (see Figure 18). The reactive dibenzylcyclooctyne (DBCO) functionality was attached by i) amidation of PLGA with NH₂-PEG-COOH via NHS ester activation and subsequent conversion with DBCO amine again by NHS ester activation. The peptides can then be attached via a Click reaction (azide-alkyne or thiol-maleimide Click) (see Figure 19).

### Formulation and characterization of reactive nanoparticles for minibinder immobilization

A first set of 8 different particles (Table 1) of similar size was formulated from (PEG-)maleimide-modified and unmodified PLGA as a mixture or pure using the single emulsion technique with polyvinyl alcohol (PVA) as a surfactant. The nanoparticles were incubated with LCB1 in presence of the reducing agent tris(2-carboxyethyl)phosphine (TCEP) in order to achieve covalent binding of LCB1 at the nanoparticles' surface. Particle S was included as a control to check whether non-covalent binding of LCB1 to the particles may occur.

**Table 1: Set of virus-binding-conjugates (VBK) loaded with LCB1.**

| **Set_1 of VBKs** | **Polymers** | **Polymer ratio** | **du /nm** ^{a} | **PDI** ^{b} |
|---|---|---|---|---|
| M | PLGA-Mal | 1 | 553 | 0.18 |
| N | PLGA-PEG-Mal | 1 | 499 | 0.15 |
| O | PLGA-Mal / PLGA | 1:1 | 523 | 0.21 |
| P | PLGA-PEG-Mal / PLGA | 1:1 | 493 | 0.23 |
| Q | PLGA-Mal / PLGA | 1:9 | 515 | 0.25 |
| R | PLGA-PEG-Mal / PLGA | 1:9 | 475 | 0.19 |
| S | PLGA | 1 | 540 | 0.21 |
| T | PLGA-Mal / PLGA-DY550 ^{c} | 1:1 | 604 | 0.27 |

| | | | | |
|---|---|---|---|---|
| **a)** Average diameter of the particles after modification with LCB1 determined by DLS. **b)** Polydispersity index determined by DLS. **c)** DY550 is a fluorescent dye to allow visualization by FACS or confocal microscopy. | | | | |

For the quantification of minibinder bound to the polymer particles, experiments were performed using the Pierce^{®} Coomassie (Bradford) Protein Assay Kit (Thermo Fisher Scientific Inc.) and the Micro BCA^{®} Protein Assay Kit (Thermo Fisher Scientific Inc.). None of these assays, however, showed conclusive results. The residual PVA from the formulation interfered with the Bradford assay. Therefore, the amount of PVA of each formulation was subsequently determined *via* PVA assay. Due to the sensitivity of the Bradford assay to detergents and the residual PVA amount of the nanoparticle suspensions, a new detergent compatible Bradford assay kit (Pierce^{®} Detergent Compatible Bradford Assay Kit (Thermo Fisher Scientific Inc.)) was used for all upcoming Bradford assays. Still the results of this assay did not show any conclusory results. Similar to this, the remaining TCEP used during the formulation to reduce the disulfide bonds affected the results of the BCA assay. Also, the use of a reducing agent compatible Pierce^{®} BCA Protein Assay Kit - Reducing Agent Compatible (Thermo Fisher Scientific Inc.) to overcome the described issues did not result in conclusive results. In consequence for the next formulations, TCEP as a reducing agent and re-buffering of LCB 1 during formulation were therefore omitted by using 50 mM HEPES, 150 mM NaCl, MilliQ water as solvent for the LCB1 protein from the beginning.

In addition, attempts were made to visualize and quantify the surface-bound proteins by SDS-PAGE. Due to their size, the particle-bound proteins are unable to migrate through the gel in the electrical field. Surface-absorbed LCB1, on the other hand, could be separated from the particles and identified by comparison with LCB1 used for formulation. Despite efforts to quantify this amount by measuring fluorescence intensity using image processing, no exact amount could be determined due to the low concentration of the protein. Nevertheless, a change in the mass of LCB1 and later LCB 1-azide was observed after maleimide-thiol and azide-alkyne reaction, respectively, with a small water-soluble polymer, which appears to confirm the reaction itself.

### Flow cytometry analysis of virus-binding conjugates (VBK) in the presence of fluorescently labeled SARS-CoV-2 RBD

In order to quantify the amount of bound LCB 1, the particles M-S were incubated with a recombinant wt-SARS-CoV-2 RBD fluorescently labeled with the Alexa Fluor 488 dye (RBD_{AF488}) and then analyzed using flow cytometry. The spectra as obtained show the comparison of the detected signals of unstained VBK to its stained analog. While the particles M-R showed no fluorescence when not pre-incubated with RBD_{AF488}, the distribution of fluorescence intensity was shifted to higher intensities after incubation with RBD_{AF488} as expected. The strongest shift was observed for VBK_P (PLGA-PEG-Mal/PLGA, 5:5). Around 88% of all particles P treated with RBD_{AF488} showed a higher fluorescence intensity than the non-treated ones. In contrast, the particles S (composed of PLGA only) which are not able to covalently bind LCB 1, only showed a slightly broadened distribution of fluorescence intensity when pre-incubated with RBD_{AF488} compared with non-treated particles.

### Virus neutralization with minibinder-functionalized particles

In order to assess whether the VBKs were able to inhibit the infection of Vero cells, replication-defective spike-pseudotyped Vesicular stomatitis virus (strain *Indiana*; VSVΔG-S) virions displaying the spike protein of the delta-variant (B.1.617.2) on their surface were produced. Because of the spike proteins on their surface, these virions are able to infect cells displaying ACE2 receptors on their surface similar to SARS-CoV-2 [11]. The genome of the VSVΔG-S virions employed here is deleted of the VSV surface protein G but instead contains a gene encoding for green fluorescent protein (GFP) allowing for a fluorescence-based read-out of viral infection. Neutralization of the used VSVΔG-S virions by the different virus-binding conjugates (VBK) resulted in reduced uptake by Vero cells and in consequence, a reduced number of GFP-positive cells after co-incubation. The half-maximal inhibitory concentration (IC₅₀) of each VBK was determined by incubating the VSVΔG-S with serially diluted VBKs for 1 h and subsequent inoculation of Vero cells (Figure 4). After 16 h of co-incubation, the GFP-positive cells were counted. While all particles were found to reduce the infection by VSVΔG-S virions, particles P and S are most effective. This inhibitory activity of the VBKs was specific for Spike-bearing virions as infection of VSVΔG particles pseudotyped with the VSV endogenous surface protein G (VSVΔG-G; as negative control) was not impacted by the VBKs.

### Neutralizing effect of VBKs on live SARS-CoV-2 in vitro

After it could be shown that the VBKs were able to reduce the infection due to VSVΔG-S virions, their antiviral effect on "live" SARS-CoV-2 was assessed in an assay relying on the human lung-cancer-derived Calu-3 epithelial cell line. Prior to testing the neutralizing ability of the VBKs M-T, the particles' toxicity and their effect on Calu-3 cell viability were assessed. Toxicity of particles on Calu-3 cells was measured by the release of lactate dehydrogenase (LDH). In comparison to non-treated Calu-3 cells (negative control), all tested particles showed no significant effect on the integrity of the cell membrane after 48 hours of incubation (Figure 5), suggesting no toxicity of these VBKs.

The particles' effect on cell viability was measured by observing the reduction of yellowish 3-(4,5-dimethylthiazol-2-yl)-2,5-diphenyltetrazolium bromide (MTT) to purple formazan, which is directly proportional to the metabolic activity of cells in a culture. The MTT assay showed no decrease in metabolic activity after 48 h of exposure of cells to VBKs, compared with an untreated control.

In contrast to the VSV-based infection assay, which relied on pre-incubation of the virions with the nanoparticles, the infection assay involving a clinical isolate of SARS-CoV-2 (Wuhan-like HH01 strain) was performed in a therapeutic approach. Calu-3 cells were infected with SARS-CoV-2 for 45 min before adding a single dose of VBKs (10 µg/ml). The whole experimental setup includes untreated Calu-3 cells infected with SARS-CoV-2 as negative control as well as a positive control with cells pretreated with the antiviral inhibitor Remdesivir (Remd). Quantification of infectious virus particles in the supernatant 48 hours post infection was performed by plaque test. At the used concentration of 10µg/ml, the particles M, Q, and T did not neutralize SARS-CoV-2, while particles N, O, P, R and S showed a strong reduction of virus titers after two days by several orders of magnitude (Figure 7, left panel). To check for specificity, all VBKs were tested against SARS-CoV (Figure 7, middle panel) and the related Middle East respiratory syndrome corona virus (MERS-CoV; Figure 7, right panel) in a setup identical to the infection assay described before. However, the VBKs were completely inactive against SARS-CoV and MERS-CoV, further indicating the specificity of particles modified with the minibinders for SARS-CoV-2.

To assess the antiviral activity of the VBKs N, O, P, R and S in more, the SARS-CoV-2 infection assay was repeated, albeit with varying concentrations of particles in the range between 1 pg/ml and 10 µg/ml to determine their respective inhibitory concentrations 50 and 90 (IC₅₀ and IC₉₀, respectively). Surprisingly, in this assay, the particles N, O, and R showed no activity, not even at the highest dose of 10 µg/ml - a concentration at which they were previously shown to be active (Figure 7). This may be due to reduced stability of VBKs N, O and R at 4 °C. In contrast, compounds P and S reduced the viral titers at 10 µg/ml, but not at 1 µg/ml or lower. Repetition of the assay with an adjusted concentration range of compound P between 1 µg/ml and 10 µg/ml resulted in an IC₅₀ and IC₉₀ of 4.86 µg/ml and 4.99 µg/ml, respectively, suggesting a narrow range of therapeutic activity which is also reflected in the steep hill slope of the non-linear regression curve.

### Phagocytosis of virus-nanoparticle complexes

Besides the efficient reduction of viral titers by simply blocking the viral spike RBDs, phagocytosis of SARS-CoV-2-nanoparticle complexes is a key invention in our project. Murine RAW264.7 macrophages were therefore either incubated with fluorescently labelled VSVΔG-S (VSVΔG-S_{Atto643}) virions alone or co-incubated with VSVΔG-S_{Atto643} virions and particles P (covalent bounded minibinder) or S (adsorbed minibinder) to check if the particles triggered viral aggregation and induced their phagocytosis. Prior to infection, RAW264.7 cells were prestained with Cholera Toxin subunit B Alexa Fluor 555 (CTB-AF555) to visualize the sphingolipid GM1. Sphingolipids like GM1 are part of the cell membrane as well intracellular membranes and enriched in lipid raft microdomains, which play an important role in phagosomal maturation [12]. Phagocytosis events were monitored using confocal laser scanning microscopy (CLSM) after a period of two or four hours. Infection of RAW264.7 macrophages with VSVΔG-S_{Atto643} virions alone showed a predominant association of virions attached to the cell membrane. Uptake of the virions by these macrophage-like cells was observed just occasionally (Figure 9). An infection of RAW264.7 macrophages with VSVΔG-S_{Atto643} and VBK_P resulted, especially after four hours of co-incubation in an increased internalization of virions and the association of some virions with the cell membrane was still present. In contrast, an infection with VSVΔG-S_{Atto643} and particles of VBK_S results in less and similar uptake like the VSVΔG-S_{Atto643} control. The quantification is based on a co-localization of signals of VSVΔG-S_{Atto643} with the intracellular signal of the CTB-AF555 staining.

In the context of the present invention, two minibinders, LCB 1 and AHB2 were successfully produced in *E. coli,* purified and analyzed regarding their affinity to RBDs of various SARS-CoV-2 variants. Even though LCB 1 was reported to have a higher affinity for the wt-spike RBD than AHB2, the inventors also included AHB2 in their investigations, because the two minibinders differ in their structure which may affect the binding affinity to other variants of concern of SARS-CoV-2 [8]. While LCB1 was designed *de novo,* AHB2 is based on helix 1 of hACE2, which constitutes a major part of the RBD-binding interface of hACE2 [8]. Resulting from the *de novo* design process, LCB1 possesses a binding site different from that of hACE2, even though both bind the same surface of the viral spike RBD. The differences between LCB 1 and hACE2 could lead to the evolution of viral escape mutants that bind hACE2 but not LCB 1. Since AHB2 and hACE2 have a similar binding site, the evolution of escape mutants that bind hACE2 but not AHB2 is less likely. As expected, the affinity of LCB 1 for the omicron RBD was reduced by two orders of magnitude compared to the wt-RBD. By contrast, the stability of the omicron RBD·AHB2 complex is only reduced by approximately one order of magnitude compared to that of the wt-RBD·AHB2 complex. Still, their affinity for the omicron RBDs is comparable. In conclusion, LCB 1 was preferred over AHB2, because it could be produced in higher yields and is smaller in size while displaying a similar affinity for the heavily mutated omicron RBDs.

Regarding the size of the nanoparticles as used, several factors were considered. First of all, internalization efficiency of spherical nano- and microparticles by cells is size-dependent [13], [14]. However, depending on the particle size, uptake of particles by cells may lead to a pro-inflammatory immune response [15]. Another aspect of the particle size to be considered is the requirement that particles penetrate the mucus layer of the airway surface liquid in the lung because we envision the particles to be administered by inhalation. Particles of 200 nm in diameter or smaller are able to diffuse through the mucus, while those with a 500 nm increased diameter or larger apparently cannot [16]. However, it should be noted that particles formulated with PEG-PLGA like in this invention showed an increased mucus diffusion. It could be demonstrated that nanoparticles with a diameter of 200 to 500 nm were able to rapidly diffuse through a human mucus layer [17].

For the approach here, quantitative detection of particles by CLSM was crucial, and particles smaller than 500 nm in diameter are very hard to detect with this technique. Consequently, a set of eight different particles with an average diameter of approximately 500 nm was formulated. This size is a compromise between mucus permeability, efficient phagocytosis and detectability by CLSM. The set of particles was formulated with different mixtures of thiol-reactive and inert PLGA to tune the density of maleimide moieties at the particle's surface.

Determining the amount of minibinder attached to the particles turned out to be rather difficult. Neither the BCA or the Bradford assay, nor the analysis by SDS-PAGE led to reliable results. However, analysis by flow cytometry of VBKs clearly revealed that only the particles S, which are unable of covalently binding the minibinder, bound insufficiently to the fluorescent SARS-CoV-2 spike RBD. By contrast, the other particles M through R bound the spike RBD, as indicated by the signal shift correlated with an increase of fluorescence intensity.

Surprisingly, the number of thiol-reactive groups incorporated into the VBKs and *ipso facto* the amount of covalently bound minibinder did not correlate with the results of the viral inhibition assay using pseudotyped VSVΔG-S. Apart from the particles P (which comprise a PLGA core, a PEG linker and covalently bound minibinder) the particles S (which do not carry covalently bound minibinders) were shown to be most active in the VSV-based assay. By contrast, the particles M which contain the highest amount of maleimide groups and could therefore display most minibinders on their surface had a very low VSVΔG-S inhibition efficacy. The antiviral activity of particle S can be explained by presence of a substantial amount of minibinder adsorbed to the particle surface in a non-covalent binding. In the virus inhibition assay, the adsorbed minibinder is assumed to be released into the medium where it blocks (covers) the viral spike protein.

The results of the SARS-CoV-2 inhibition assays are comparable to those of the VSV-based inhibition assay, making the latter a good approximation of SARS-CoV-2 inhibition. This is in line with a previous study which found that a pseudotyped VSV-based assay delivers results comparable to a viral inhibition assay involving a clinical isolate of SARS-CoV-2 [18]. In the present SARS-CoV-2 neutralization assay, not only particles M and T but also particles Q were unable to inhibit SARS-CoV-2 infection of Calu-3 cells at particle concentrations of 10 µg/ml. One aspect in this context is that a PEG linker is essential for the flexibility of the attached minibinders, which is required to allow for the correct orientation of the minibinders to bind the viral spike RBD. However, particles S have no PEG-linker on their surface but are able to inhibit SARS-CoV-2 infection of Calu-3 cells. In a repetition of the experiments, also the particles N, O and R were shown to be inactive against SARS-CoV-2 at 10 µg/ml. For those particles the concentration of 10 µg/ml might be below the threshold of activity and minor variations in the VBK concentration or in the initial viral concentration therefore have a strong influence whether activity is observed or not. The half-maximal inhibitory concentration of VBK_P was determined to be approximately IC₅₀ = 4.9 µg/ml and the IC₅₀ of compound S was observed to be in the same order of magnitude, i.e., between 1 µg/ml and 10 µg/ml. When applied to the same assay, the free minibinder LCB 1 was observed to have an IC₅₀ between 10 nM and 100 nM.

As expected from the high specificity of the minibinder with respect to the spike RBD of SARS-CoV-2 [8], inhibition of SARS-CoV and MERS-CoV by the VBKs M through T was not observed. Further, the free minibinder LCB 1 as well as the particles M through T were found to have no toxicity with respect to Calu-3 epithelial cells. It should be noted, that the virus neutralization assays (VSV-based and SARS-CoV-2-based) only inform on the ability of the employed particles to block the spike RBD, but do not provide information on the formation of viral aggregates that would facilitate phagocytosis by macrophages. Plausible modes of action of the VBKs include (1) the release of previously adsorbed minibinders into the medium to block the spike RBD and/or (2) interaction of the VBKs with virions which blocks their spike RBD and compromises their ability to enter the host cells.

To assess whether the VBKs could elicit phagocytosis of virions, they were applied in a phagocytosis model involving murine RAW264.7 macrophages and VSVΔG-S_{Atto643} virions. As those virions cannot proliferate, they are a harmless surrogate for the highly infectious SARS-CoV-2 and can be handled without the need of biosafety level 3 facilities [18]. With dimensions of approximately 220 nm (length) by 83 nm (diameter) [19] the bullet-shaped VSVΔG-S_{Atto643} virions are only slightly larger than SARS-CoV-2 virions. In addition to their similar size, they display SARS-CoV-2-derived spike proteins on their surface and are therefore well-suited to represent SARS-CoV-2 virions in the phagocytosis assay to elucidate the cellular mode of action of the VBKs. Remarkably, when murine RAW264.7 macrophages were infected with VSVΔG-S_{Atto643} virions, the presence of particles P significantly increased the number of phagocytosis events observed, compared to a control experiment in which no VBKs were present, thus fully supporting our initial hypothesis. It is very likely that the number of phagocytosis events would further increase, if macrophages were used that display the human ACE2 receptor on their surface for efficient binding of SARS-CoV-2 aggregates. The low titers of VSVΔG-S_{Atto643} virions (multiplicity of infection = 0.1) employed here is also a crucial factor in *in vitro* studies which limits the evaluation of different infection conditions, like infection dose or ratio of virion to VBKs. Despite these limitations in the assay employed here, it was not only possible to show neutralization of virions in the presence of VBKs, but the inventors also provided evidence of an increased uptake of virions by immune cells.

### Literature as cited

1. Lu, R., et al., Genomic characterisation and epidemiology of 2019 novel coronavirus: implications for virus origins and receptor binding. Lancet, 2020. 395(10224): p. 565-574.
2. Lan, J., et al., Structure of the SARS-CoV-2 spike receptor-binding domain bound to the ACE2 receptor. Nature, 2020. 581(7807): p. 215-220.
3. Zhu, N., et al., A Novel Coronavirus from Patients with Pneumonia in China, 2019. N Engl J Med, 2020. 382(8): p. 727-733.
4. World Health Organization, Tracking SARS-CoV-2 variants, 01.02.2023. https://www.who.int/activities/tracking-SARS-CoV-2-variants/.
5. Aderem, A. and D.M. Underhill, Mechanisms of phagocytosis in macrophages. Annu Rev Immunol, 1999. 17: p. 593-623.
6. Patel, M., et al., The Immunopathobiology of SARS-CoV-2 Infection. FEMS Microbiol Rev, 2021. 45(6).
7. Bar-On, Y.M., et al., SARS-CoV-2 (COVID-19) by the numbers. Elife, 2020. 9.
8. Cao, L., et al., De novo design of picomolar SARS-CoV-2 mini protein inhibitors. Science, 2020. 370(6515): p. 426-431.
9. Shkodra-Pula, B., et al., Effect of surfactant on the size and stability of PLGA nanoparticles encapsulating a protein kinase C inhibitor. Int J Pharm, 2019. 566: p. 756-764.
10. Knop, K., et al., Poly(ethylene glycol) in drug delivery: pros and cons as well as potential alternatives. Angew Chem Int Ed Engl, 2010. 49(36): p. 6288-308.
11. Schmidt, F., et al., Measuring SARS-CoV-2 neutralizing antibody activity using pseudotyped and chimeric viruses. J Exp Med, 2020. 217(11).
12. Schmidt, F., et al., Flotillin-Dependent Membrane Microdomains Are Required for Functional Phagolysosomes against Fungal Infections. Cell Rep, 2020. 32(7): p. 108017.
13. Leclerc, L., et al., Size of submicrometric and nanometric particles affect cellular uptake and biological activity of macrophages in vitro. Inhal Toxicol, 2012. 24(9): p. 580-8.
14. Pacheco, P., D. White, and T. Sulchek, Effects of microparticle size and Fc density on macrophage phagocytosis. PLoS One, 2013. 8(4): p. e60989.
15. Yue, H., et al., Particle size affects the cellular response in macrophages. Eur J Pharm Sci, 2010. 41(5): p. 650-7.
16. Schuster, B. S., et al., Nanoparticle diffusion in respiratory mucus from humans without lung disease. Biomaterials, 2013. 34(13): p. 3439-46.
17. Huckaby, J.T. and S.K. Lai, PEGylation for enhancing nanoparticle diffusion in mucus. Adv Drug Deliv Rev, 2018. 124: p. 125-139.
18. Case, J.B., et al., Neutralizing Antibody and Soluble ACE2 Inhibition of a Replication-Competent VSV-SARS-CoV-2 and a Clinical Isolate of SARS-Coll 2. Cell Host Microbe, 2020. 28(3): p. 475-485 e5.
19. Ge, P., et al., Cryo-EM model of the bullet-shaped vesicular stomatitis virus. Science, 2010. 327(5966): p. 689-93.
20. Wan, Y., et al., Receptor Recognition by the Novel Coronavirus from Wuhan: an Analysis Based on Decade-Long Structural Studies of SARS Coronavirus. J Virol, 2020. 94(7).
21. Pharo, E.A., et al., Host-Pathogen Responses to Pandemic Influenza H1N1pdm09 in a Human Respiratory Airway Model. Viruses, 2020. 12(6).
22. Bordoni, V., et al., SARS-CoV-2 Infection of Airway Epithelium Triggers Pulmonary Endothelial Cell Activation and Senescence Associated with Type I IFN Production. Cells, 2022. 11(18).
23. Sia, S.F., et al., Pathogenesis and transmission of SARS-CoV-2 in golden hamsters. Nature, 2020. 583(7818): p. 834-838.
24. Imai, M., et al., Syrian hamsters as a small animal model for SARS-CoV-2 infection and countermeasure development. Proc Natl Acad Sci USA, 2020. 117(28): p. 16587-16595.
25. Li, L., et al., Structural basis of human ACE2 higher binding affinity to currently circulating Omicron SARS-CoV-2 sub-variants BA.2 and BA.1.1. Cell, 2022. 185(16): p. 2952-2960 e10.
26. Hunt, A.C., et al., Multivalent designed proteins neutralize SARS-Coll2 variants of concern and confer protection against infection in mice. Sci Transl Med, 2022. 14(646): p. eabn1252.
27. Chevalier, A., et al., Massively parallel de novo protein design for targeted therapeutics. Nature, 2017. 550(7674): p. 74-79.
28. Case, J.B., et al., Ultra potent miniproteins targeting the SARS-CoV-2 receptor-binding domain protect against infection and disease. Cell Host Microbe, 2021. 29(7): p. 1151-1161 e5.

## Claims

1. A functionalized nano- or microparticle for use in the prevention and/or treatment of target virus infection in a mammalian subject, comprising,
a) at least one nano- or microparticle, and
b) at least one virus-binding peptide and/or virus-binding small molecule immobilized onto the surface of the nano- or microparticle, wherein the virus-binding peptide and/or a virus-binding small molecule binds to the at least one target virus with nano- to picomolar affinity, and
wherein the functionalized nano- or microparticle has an overall diameter of at least about 400 nm, preferably between about 400 to 2000 nm, and more preferably between about 600 and 700 nm,
wherein preferably said binding between the virus-binding peptide and/or a virus-binding small molecule and the at least one target virus outcompetes binding of the target virus to its host cell receptor.

2. The functionalized nano- or microparticle for use according to claim 1, wherein said functionalized nano- or microparticle is substantially spherical in shape.

3. The functionalized nano- or microparticle for use according to claim 1 or 2, wherein said nano- or microparticle comprises a suitable organic or inorganic material, such as, for example, a metal, a plastic, a polymer, such as, for example, PLA, PLGA, PLGH, polyglutamate, styrene-maleic acid copolymer or PE, or is a nanosphere composed of membranes and/or lipids, such as a liposome, preferably wherein said nano- or microparticle comprises PLGA-Mal, PLGA-PEG-Mal, PLGA-DY550, PLGA-PEG, PLGA-PEG-DBCO, and/or combinations thereof.

4. The functionalized nano- or microparticle for use according to any one of claims 1 to 3, wherein the virus-binding peptide is selected from a minibinder protein, a host cell receptor, sialylated glycans such as SAs and SA-containing gangliosides, cell adhesion molecules (CAMs), such as igSF members including CD4, JAM-A, CAR, integrins, such as αvβ3, PtdSer receptors, cell immunoglobulin and mucin domain (TIM), Tyro3, Axl, Mer (TAM), and ACE2 receptor, wherein preferably the virus-binding peptide is selected from LCB 1, and AHB2.

5. The functionalized nano- or microparticle for use according to claim 4, wherein said virus-binding peptide comprises at least one terminal cysteine residue, said cysteine residue is optionally modified to carry an azide group.

6. The functionalized nano- or microparticle for use according to any one of claims 1 to 5, wherein said immobilization of the virus-binding peptide or virus-binding small molecule to the nano- or microparticle surface is direct or indirect, for example through conjugation, physical adsorption and/or covalent linkage, wherein preferably the immobilization is a succinimidyl thioether bond or a bibenzocyclooctyne (DBCO) azide ligation.

7. The functionalized nano- or microparticle for use according to any one of claims 1 to 6, wherein said functionalized nano- or microparticle further comprises at least one antiviral drug.

8. The functionalized nano- or microparticle for use according to any one of claims 1 to 7, wherein said functionalized nano- or microparticle further comprises at least one detectable label or marker.

9. An aggregate comprising at least one functionalized nano- or microparticle for use according to any one of claims 1 to 8 and at least one target virus for use in the prevention and/or treatment of target virus infection in a mammalian subject, wherein said prevention and/or treatment comprises phagocytosis, in particular cell-mediated phagocytosis in said mammalian subject.

10. The functionalized nano- or microparticle for use according to any one of claims 1 to 8 or the aggregate for use according to claim 9, wherein said at least one target virus is selected from the group of HIV, measles virus, reovirus, rhinovirus, adenovirus, poliovirus, coxsackievirus B, reovirus, rotavirus, adenovirus, West Nile virus, human metapneuomovirus (hMPV), foot-and-mouth disease virus (FMDV), herpes simplex virus (HSV), HPV, human cytomegalovirus HCMV, human herpesvirus-8, rabies virus, hMPV, paramyxovirus, filoviruses, Ebola virus (EBOV), Marburgvirus (MARV), flaviviruses, dengue virus (DENV), Zika virus (ZIKV), Lassa Virus, poxvirus, SV40, BKPyV, PyV, Influenza A virus (IAV), Orthomyxoviridae, MERS-CoV, betacoronavirus, SARS-S, and SARS-CoV-2.

11. A pharmaceutical composition comprising the functionalized nano- or microparticle for use according to any one of claims 1 to 8 or 10 or the aggregate for use according to claim 9 or 10 for use in the prevention and/or treatment of target virus infection in a mammalian subject, wherein said pharmaceutical composition additionally comprises suitable excipients and/or auxiliary agents.

12. The pharmaceutical composition for according to claim 11, wherein said functionalized nano- or microparticle has sizes between about 200 to 500 nm and is optionally suitable for administration through inhalation.

13. A method for detecting a target virus and/or target virus specific anti-virus antibodies in a biological sample obtained from a mammalian subject, comprising contacting the functionalized nano- or microparticle for use according to any one of claims 1 to 8 with said biological sample, and detecting binding of said target virus and/or target virus specific anti-virus antibodies to said particle and/or detecting forming of aggregates of said target virus with said functionalized nano- or microparticle, wherein a binding and/or forming of aggregates is indicative for the target virus and/or target virus specific anti-virus antibodies in the biological sample, wherein preferably said functionalized nano- or microparticle further comprises at least one detectable label or marker, and wherein further preferably the sample is a blood or serum sample derived from the mammal, or a pooled sample from a group of subjects.

14. The method according to claim 13, further comprising the step of quantifying the amount of a target virus and/or target virus specific anti-virus antibodies in the biological sample obtained from the mammalian subject based on the binding and/or forming of aggregates in the biological sample.

15. A kit for performing the method according to claim 13 or 14, comprising at least one functionalized nano- or microparticle for use according to any one of the claims 1 to 8, together with suitable auxiliary agents.
